# EUROPEAN PATENT SPECIFICATION

(11) **EP 4 426 298 B1**
(45) Date of publication and mention of the grant of the patent: **11.02.2026**
(21) Application number: 22839143.9
(22) Date of filing: 08.12.2022
(51) Int. Cl.: A61K 31/4418, A61K 31/496, A61K 31/519, A61P 11/00

(54) **NEW THERAPEUTIC COMBINATIONS COMPRISING PIRFENIDONE AND PDE4-INHIBITOR FOR THE TREATMENT OF PROGRESSIVE FIBROSING INTERSTITIAL LUNG DISEASES**
NEUE THERAPEUTISCHE KOMBINATIONEN ENTHALEND PIRFENIDON UND PDE4-INHIBITOR ZUR BEHANDLUNG VON FORTSCHREITENDEN FIBROSIERENDEN INTERSTITIELLEN LUNGENERKRANKUNGEN
NOUVELLES COMBINAISONS THÉRAPEUTIQUES CONTENAT DE LA PIRFÉNIDONE ET DE L'INHIBITEUR DE PDE4 POUR LE TRAITEMENT DE MALADIES PULMONAIRES INTERSTITIELLES FIBROSANTES PROGRESSIVES

(30) Priority: 09.12.2021 US 202163287641 P; 29.12.2021 EP 21218207; 08.06.2022 EP 22177757
(43) Date of publication of application: 11.09.2024
(60) Divisional application: 24215043.1
(73) Proprietor: Boehringer Ingelheim International GmbH, 55216 Ingelheim am Rhein (DE)
(72) Inventor: HESSLINGER, Christian, 55216 Ingelheim am Rhein (DE); BAUER, Verena, 55216 Ingelheim am Rhein (DE); BOSSERT, Sebastian Martin, 55216 Ingelheim am Rhein (DE); KOBER, Susan, 55216 Ingelheim am Rhein (DE); LIU, Yi, Ridgefield, Connecticut 06877-0368 (US); NICKOLAUS, Peter, 55216 Ingelheim am Rhein (DE); SARNO, Maria, 55216 Ingelheim am Rhein (DE); VOSS, Florian, 55216 Ingelheim am Rhein (DE)
(74) Representative: Lutze, Oliver
(86) International application number: PCT/EP2022/084972
(87) International publication number: WO 2023/104961

(56) References cited:
- WO-A1-2019/081235
- WO-A1-2021/198253

## Description

### 1. Background of the Invention

### 1.1 Phosphodiesterases and their role in fibrosis

Phosphodiesterases (PDEs) mediate the hydrolysis of the second messengers, cyclic adenosine monophosphate (cAMP) or cyclic guanosine monophosphate. PDEs are coded by 11 gene superfamilies containing multiple genes (coding for subtypes A, B, C, etc.) that also give rise to alternative mRNA-splicing variants leading to approximately 100 PDE isoforms. PDE4 has traditionally been implicated in the regulation of inflammation and the modulation of immunocompetent cells, and the three selective PDE4 inhibitors currently available support a beneficial role for PDE4 inhibitors in inflammatory and/or autoimmune diseases (Sakkas et al., 2017, Curr. Med. Chem. 24, 3054-3067; Li et al., 2018, Front. Pharmacol. 9, 1048). The first-in-class PDE4 inhibitor, oral roflumilast (Daliresp^{®}, Daxas^{®}), was approved by the U.S. Food and Drug Administration in 2011 to reduce the risk of COPD exacerbations in patients with severe COPD associated with chronic bronchitis and a history of exacerbations (U.S. Food & Drug Administration, 2013, DALIRESP^{®} (roflumilast)). Another compound, oral apremilast (Otezla^{®}), was approved for the treatment of psoriatic arthritis and plaque psoriasis in 2014 (U.S. Food & Drug Administration, 2017, OTEZLA^{®} (apremilast)). A third PDE4 inhibitor, crisaborole (Eucrisa^{®}), was approved in 2016 for topical treatment of mild-to-moderate atopic dermatitis (U.S. Food & Drug Administration, 2016, EUCRISA^{™} (crisaborole)). None of these show any preferential enzymatic inhibition among the four PDE4 subtypes, A-D.

The general anti-inflammatory potential of PDE4 inhibition, as exemplified by roflumilast, is well established (Hatzelmann et al., 2010, Pulm. Pharmacol. Ther. 23, 235-256), and the use of PDE4 inhibitors in various inflammatory and immune-mediated diseases has been broadly investigated (Sakkas et al., 2017, Curr. Med. Chem. 24, 3054-3067; Li et al., 2018, Front. Pharmacol. 9, 1048). However, in the last decade, it has become increasingly clear that PDE4 may also play an important role in fibrosis, based on animal studies and on in vitro experiments evaluating the functional role of PDE4 inhibitors in fibroblasts. The attenuation of lung fibrosis by PDE4 inhibitors has been demonstrated under various experimental conditions, most widely in bleomycin-induced fibrosis in rodents. In rat models, rolipram was shown to inhibit fibrotic score, hydroxyproline content, and serum tumor necrosis factor-a (TNF-α) (Pan et al., 2009, Respirology 14, 975-982). In this initial study, the PDE4 inhibitor was administered from the beginning of bleomycin challenge, so it was not clear whether rolipram was primarily active due to inhibition of initial inflammation or inhibition of secondary fibrosis. A second early study in mice and rats, however, showed that oral roflumilast was active both in preventive and in therapeutic protocols in a dose-dependent manner (Cortijo et al., 2009, Br. J. Pharmacol. 156, 534-544). In lung extracts, roflumilast inhibited histologically assessed fibrosis, hydroxyproline content, and the mRNA expression of TNF-α, transforming growth factor-β (TGF-β), connective tissue growth factor (CTGF), α1 collagen, endothelin-1, and mucin 5ac. In bronchoalveolar lavage fluid (BALF), the levels of TNF-α, interleukin (IL)-13, TGF-β, and mucin 5ac, the formation of lipid hydroperoxides, and the influx of inflammatory cells (e.g. neutrophils and macrophages) were inhibited. Besides fibrosis, right ventricular hypertrophy and vascular remodeling (pulmonary arteries) were positively influenced by roflumilast. The same group later on also demonstrated that the metabolome associated with pulmonary fibrosis in bleomycin mice was modulated by roflumilast. Levels of the amino acids (AAs) glycine and proline, involved in collagen formation/structure, were lowered by roflumilast, while lung glutathione and plasma tetrahydrobiopterin were increased, suggesting an alteration in oxidative equilibrium by roflumilast (Milara et al., 2015, PLoS One 10, e0133453). Another PDE4 inhibitor, cilomilast, was shown to inhibit late-stage lung fibrosis and tended to reduce collagen content in bleomycin mice, although no effect on TGF-β1 and col1agen type (Col) 1A1 expression was found (Udalov et al., 2010, BMC Pulm. Med. 10, 26).

Improvement of lung fibrosis by PDE4 inhibition was not limited to the bleomycin model. In a murine model of lung fibrosis targeting type II alveolar epithelial cells in transgenic mice expressing the diphtheria toxin receptor under the control of the murine surfactant protein C promoter, roflumilast lowered lung hydroxyproline content and mRNA expression of TNF-α, fibronectin (FN), and CTGF (Sisson et al., 2018, Physiol. Rep. 6, e13753). Interestingly, roflumilast was active both in a preventive and in a therapeutic regimen, and under the latter conditions appeared to be therapeutically equipotent to pirfenidone and nintedanib. Furthermore, in a mouse model of chronic graft-versus-host disease, lung fibrosis was attenuated by oral roflumilast (Kim et al., 2016, Exp. Hematol. 44, 332-341.e334). roflumilast inhibited fibrosis, collagen deposition, hydroxyproline and TGF-β1 content, cell infiltration, and expression of mRNA for IL-6 and IL-1β. In addition, in the BALF inflammatory cells (macrophages, lymphocytes, neutrophils, and eosinophils), expression of mRNA for IL-6, IL-1β, and monocyte chemotactic protein-1 was inhibited by roflumilast. In a rabbit tuberculosis model, pulmonary damage and fibrosis were shown to be inhibited by two PDE4 inhibitors from Celgene, CC-3052 (Subbian et al., 2011, Am. J. Pathol. 179, 289-301) and CC-11050 (Subbian et al., 2016, EBioMedicine 4, 104-114). PDE4 inhibition improved antibiotic therapy and lung fibrosis by positively influencing collagen deposition and mRNA expression of various matrix metalloproteinases, including metalloproteinase 12.

Besides the lung, beneficial effects of PDE4 inhibition on fibrosis have been demonstrated in several other organs including skin, liver, kidney, and colon. For example, in various preclinical mouse models of SSc, skin fibrosis induced by either bleomycin or topoisomerase I and chronic graft-versus-host disease was inhibited by rolipram and apremilast (Maier et al., 2017, Ann. Rheum. Dis. 76, 1133-1141). This group did not find direct inhibitory effects of PDE4 inhibition on the release of profibrotic cytokines (IL-6, IL-13, TGF-β1/β2) in fibroblasts and M2 macrophages purified from peripheral blood of patients with SSc, which may be due to the lack of an exogenous cAMP trigger under the experimental conditions used. In a model of unilateral ureteral obstruction-induced obstructive nephropathy in mice, rolipram was shown to inhibit renal interstitial fibrosis (Ding et al., 2017, Antioxid. Redox Signal. 29, 637-652). In mouse primary tubular epithelial cells in vitro, TGF-β up-regulated PDE4A/B, and rolipram inhibited TGF-β-induced damage, FN expression, and deficiency of mitochondrial biogenesis. roflumilast inhibited diethylnitrosamine-induced liver fibrosis, hydroxyproline deposition, and TGF-β1 expression in rats (Essam et al., 2019, Life Sci. 222, 245-254). Similarly, rolipram inhibited collagen deposition, α-smooth muscle actin (α-SMA) staining, and mRNA expression, as well as the expression of TGF-β1 mRNA and TNF-α protein, in a bile duct ligation-induced hepatic fibrosis model in rats, with up-regulation of PDE4A, B, and D (Gobejishvili 2019). In hepatic stellate cells in vitro, rolipram inhibited mRNA expression of α-SMA and Col1A2 (Gobejishvili et al., 2013, J. Pharmacol. Exp. Ther. 347, 80-90). With respect to colonic tissue, rolipram inhibited collagen and TGF-β1 in a model of trinitrobenzene sulfonic acid-induced colitis in rats (Videla et al., 2006, J. Pharmacol. Exp. Ther. 316, 940-945), and apremilast inhibited fibrosis in colon, collagen deposition, and the expression of genes related to fibrosis in a model of dextran sulfate sodium-induced colitis ulcerosa in mice (Li et al., 2019, Br. J. Pharmacol. 176, 2209-2226). In a murine cecal abrasion model, rolipram inhibited fibrotic reactions, indicating that PDE4 inhibition has the potential to prevent postoperative intra-abdominal adhesions (Eser et al., 2012, Dis. Colon Rectum 55, 345-350). Adhesions are assumed to result from laparotomy by abnormal healing. In support of this assumption, rolipram has been shown to be active in a subcutaneous or intraperitoneal polyether-polyurethane sponge implant model in mice by inhibiting intra-implant collagen and TGF-β1 deposition (Mendes et al., 2009, Microvasc. Res. 78, 265-271). Thus, in various animal models, the beneficial impact of selective PDE4 inhibition on fibrosis has been proven, most extensively in the lung but also in several other organs. While the specific target(s) of PDE4 inhibitors in fibrotic diseases are largely unknown, it is tempting to speculate that they act either indirectly via inhibition of pro-inflammatory cells and mediators, and/or directly by inhibiting the typical effector cells (fibroblasts, myofibroblasts) mediating fibrosis.

The direct modulation of various fibroblast functions by PDE4 inhibitors has been demonstrated in fibroblast cell lines of human origin. Kohyama et al. demonstrated a direct impact of PDE4 inhibitors on fibroblasts in vitro (Kohyama et al., 2004, Clin. Immunol. 111, 297-302). In human fetal lung fibroblasts (HFL-1), rolipram and cilomilast inhibited FN-induced chemotaxis and contraction of collagen gels. Inhibition of fibroblast function by prostaglandin E2 (PGE2) was shifted to the left in the presence of PDE4 inhibitors, and the inhibition of endogenous PGE2 by indomethacin diminished their effects (Kohyama et al., 2002, Am. J. Respir. Cell Mol. Biol. 26, 694-701). The inhibition of the fibroblast cell line HFL-1 functions by cilomilast could be modulated by cytokines like IL-1β (which up-regulated PGE2 and shifted the cilomilast curve to the left) or IL-4 (which down-regulated PGE2 and shifted the cilomilast curve to the right). The inhibition of HFL-1 functions (FN-stimulated chemotaxis and collagen gel contraction) by rolipram and roflumilast was dependent on cyclooxygenase-2 expression and subsequent PGE2 synthesis (Kohyama et al., 2002, Am. J. Respir. Cell Mol. Biol. 26, 694-701). In addition, TGF-β1-stimulated FN release was inhibited by the PDE4 inhibitors, paralleled by a stimulation of PGE2 release as a positive feedback mechanism (Togo et al., 2009, Am. J. Physiol. Lung Cell Mol. Physiol. 296, L959-L969.). In another human fetal lung fibroblast strain (GM06114), roflumilast N-oxide, the active metabolite of roflumilast, in the presence of PGE2 was shown to inhibit intercellular adhesion molecule-1 and eotaxin release stimulated by TNF-α, proliferation stimulated by basic fibroblast growth factor (bFGF) plus IL-1β, as well as TGF-β1-induced α-SMA, CTGF, and FN mRNA expression in the presence of IL-1β (Sabatini et al., 2010, Pulm. Pharmacol. Ther. 23, 283-291). Importantly, IL-1β up-regulated PDE4 activity.

In normal human lung fibroblasts (NHLFs), the impact of PDE4 inhibitors has been described in several publications. TGF-β-induced fibroblast-to-myofibroblast conversion assessed by α-SMA expression was shown to be inhibited by piclamilast in the presence of PGE2 (Dunkern et al., 2007, Eur. J. Pharmacol. 572, 12-22). In subsequent papers, the same group showed the inhibition of IL-1β plus bFGF-stimulated fibroblast proliferation by piclamilast and the importance of cyclooxygenase-2 and PGE2 (Selige et al., 2010, J. Cell. Physiol. 223, 317-326). By using PDE4 subtype-specific siRNA, the involvement of PDE4B and PDE4A in this response, as well as the involvement of PDE4B and PDE4D in TGF-β-induced α-SMA expression, were shown (Selige et al., 2011, J. Cell. Physiol. 226, 1970-1980). The importance of a cAMP trigger for the modulation of fibroblast functions by PDE4 inhibition was corroborated by the inhibition by roflumilast of TGF-β1-induced CTGF mRNA and α-SMA protein expression, and FN in the presence of the long-acting β2-adrenergic agonist indacaterol (Tannheimer et al., 2012, Respir. Res. 13, 28). In addition, various other NHLF responses (collagen synthesis, proliferation, reactive oxygen species and F2-isoprostane formation, NADPH oxidase 4 expression) stimulated by bleomycin or 8-epi-PGF2α were inhibited by roflumilast N-oxide (Vecchio et al., 2013, Mediators Inflamm. 2013, 745984). In yet another human lung fibroblast model (WI-38), TGF-β-induced mRNA expression of collagen α1, CTGF, and FN in the presence of the adenyl cyclase activator forskolin were inhibited by roflumilast and another PDE4 inhibitor (compound 1) (Sisson et al., 2018, Physiol. Rep. 6, e13753), as well as a dual-selective PDE4/5 inhibitor (compound A) (Muraki et al., 2019, Biosci. Biotechnol. Biochem. 83, 1000-1010).

Thus, a multitude of in vitro studies indicate that PDE4 inhibitors are able to directly inhibit various fibroblast functions where either an endogenous or an exogenous cAMP trigger is available in the test system. While the availability of cAMP agonists may be limited under artificial in vitro conditions, it can be expected that in diseased (inflammatory, fibrotic) tissues, cAMP agonists like PGE2, adenosine, histamine, or adrenalin may be formed. The importance of PDE4 under such conditions may be further enhanced by up-regulation of PDE4 activity by cytokines such as IL-1β.

The modulation of another interesting aspect of fibrosis, epithelial-mesenchymal transition, was addressed in the TGF-β1-stimulated A549 human alveolar epithelial cell line in vitro. TGF-β1 stimulated the up-regulation of PDE4 subtypes (PDE4A and 4D), and rolipram and siRNA inhibited epithelial-mesenchymal transition changes like FN and collagen mRNA expression, although not α-SMA mRNA (Kolosionek et al., 2009, Mol. Biol. Cell 20, 4751-4765).

### 1.2 Progressive Fibrosing Interstitial Lung Diseases (PF-ILD)

Interstitial lung diseases (ILDs) comprise a heterogeneous group of lung diseases affecting the interstitium, distinct from obstructive airway diseases such as asthma or chronic obstructive pulmonary disease (COPD). Prolonged ILD may result in pulmonary fibrosis, but this is not always the case. The most extensively studied ILD is idiopathic pulmonary fibrosis (IPF), which is characterized by progressive pulmonary fibrosis. Non-IPF ILDs may include connective tissue disease-related ILDs such as those related to rheumatoid arthritis and other autoimmune diseases, systemic sclerosis associated ILD (SSc-ILD), and polymyositis/dermatomyositis, and ILDs related to chronic sarcoidosis, chronic hypersensitivity pneumonitis, idiopathic non-specific interstitial pneumonia, and exposure-related diseases such as asbestosis and silicosis (Cottin et al, . Eur. Respir. Rev. 28, 180100; Kolb, M., and Vasakova, M. (2019), Respir. Res. 20, 57). Up to 40% of patients with these ILDs may develop a progressing fibrotic phenotype.

Progressive fibrosing ILDs are associated with high mortality, with median post-diagnosis survival in patients with IPF estimated at 2-5 years (Raghu, G., Chen, S.Y., Yeh, W.S., Maroni, B., Li, Q., Lee, Y.C., and Collard, H.R. (2014). Idiopathic pulmonary fibrosis in US Medicare beneficiaries aged 65 years and older: incidence, prevalence, and survival, 2001-11. Lancet Respir. Med. 2, 566-572). Progression of fibrosing ILD is reflected in various parameters, including decline in pulmonary function, decrease in exercise capacity, deterioration in quality of life, worsening of cough and dyspnea, acute exacerbations, and increase of morphologic abnormalities (Cottin et al. Eur. Respir. Rev. 28, 180100, 2019; Kolb and Vasakova, 2019, Respir. Res. 20, 57). In patients with IPF, forced vital capacity (FVC) is a well-established predictor of mortality, and acute exacerbations are associated with very high mortality. Although corticosteroids and/or immunosuppressive drugs are sometimes used off-label to treat progressive fibrosing ILDs, currently the only approved treatments to slow disease progression in IPF are nintedanib and pirfenidone (Richeldi et al., 2018, Eur. Respir. Rev. 27, 180074). nintedanib has been approved in the US since 2014 (U.S. Food & Drug Administration, 2020, OFEV^{®} (nintedanib)), and in Europe and Japan since 2015 (European Medicines Agency, 2021b, OFEV^{®} (nintedanib)), while pirfenidone was approved in Japan in 2008, in Europe in 2011 (European Medicines Agency, 2021a, Esbriet (pirfenidone)), and in the US in 2014 (U.S. Food & Drug Administration, 2019, ESBRIET^{®} (pirfenidone)). Lung transplantation is the only potentially curative treatment for IPF and the medical need in IPF and other progressive fibrosing ILDs remains high.

However, in patients with IPF having a mild or moderate impairment of FVC (≥50 % predicted), both presently approved medications pirfenidone and nintedanib, can only reduce the decline in FVC, consistent with a slowing of disease progression, but both are not able to stop or even reverse or heal the symptoms of IPF (Tzouvelekis et al Ther. Clin. Risk Management 2015, 11, 359-370).

Nevertheless, both treatment-options, either with pirfenidone or with nintedanib, show significant beneficial effects in slowing down IPF disease progression.

The most prominent side effects associated with both, nintedanib and pirfenidone, are gastrointestinal events, particularly diarrhea, nausea, vomiting, abdominal pain, decreased appetite and a decreased body weight. In case that gastrointestinal side effects arise, they are usually managed either by treatment interruption, dose reduction or symptomatic treatment of the gastrointestinal side effects (see Mazzei et al, Ther. Adv. Respir. Dis. 2015, Vol. 9 [3], pp. 121-129).

Due to these "accumulative gastrointestinal side effects" of pirfenidone on the one hand and of nintedanib on the other hand a combination treatment for IPF by a combination of pirfenidone and nintedanib is not frequently used. Investigations have shown that a combination treatment with pirfenidone and nintedanib leads to increased gastrointestinal side effects, in particular to diarrhoea, nausea, vomiting, and upper abdominal pain (Vancheri et al., nintedanib with Add-on pirfenidone in Idiopathic Pulmonary Fibrosis: Results of the INJOURNEY Trial. Am J Respir Crit Care Med. 2017, Epub ahead of print).

Consequently, due to the fact that both active agents which are so far approved for the treatment for IPF, pirfenidone and nintedanib, are - when administered alone - not able to stop or to heal IPF, but instead can only slow down the IPF disease progression to a certain percentage (Tzouvelekis et al Ther. Clin. Risk Management 2015, 11, 359-370), and due to the fact that additionally both nintedanib and pirfenidone show significant gastrointestinal side effects which accumulate when both compounds are combined, there is still a significant medical need for improved methods of treatment of IPF/ PF-ILD, in particular for improved methods of treatment of IPF/PF-ILD which combine an improved therapeutic efficacy (compared to standard of care treatment) with an acceptable tolerability/safety, in particular with regard to gastrointestinal side effects.

### 1.3 Patent Literature

Additionally to the approved PDE4 inhibitors roflumilast and apremilast - many further patent applications drawn on other PDE4-inhibitors with improved properties have been published:
- Pteridines as PDE4-inhibitors in WO 2006/056607, WO 2006/058869, WO 2006/058868 and WO 2006/058867.
- Piperazino-Dihydrothienopyrimidines as PDE4-inhibitors in WO 2006/ 111549, WO 2007/118793 and WO 2009/050242.
- Piperidino-Dihydrothienopyrimidines as PDE4-inhibitors in WO 2009/050248 and in WO 2013/026797.

The PDE4-inhibitors of formula I 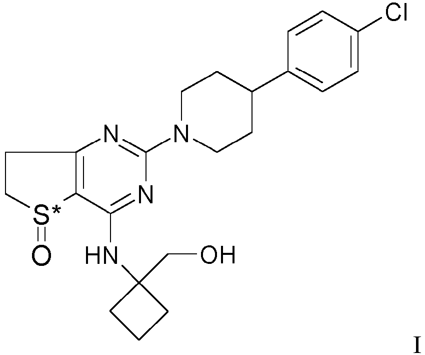 and of formula II 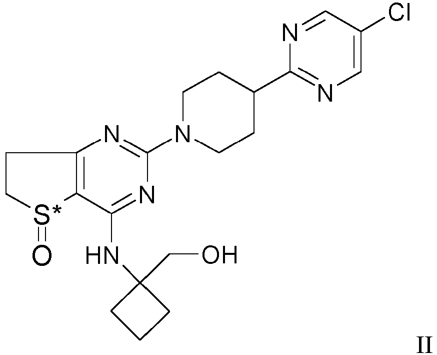 and in particular of formula III 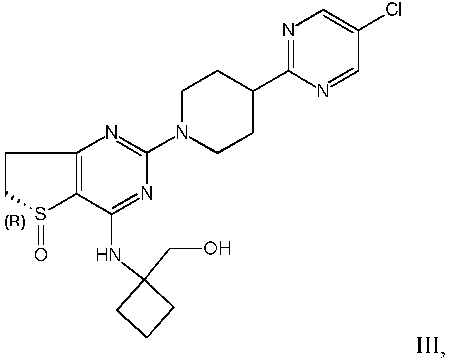
which is the R-enantiomer of the compound of formula II,
have been disclosed in WO 2013/026797. It had been shown that the PDE4-inhibitors of formulas I, II and III inhibit preferentially the PDE4 B subtype.

WO2019/081235 discloses a combination of nintedanib and of the PDE4-inhibitor of formula III which shows in an *in vitro* assay using human lung fibroblasts a synergistic overadditive effect with respect to fibroblast proliferation.

Therefore, neither WO 2013/026797 nor WO 2019/081235 disclose any doses and/or dose regimens of the PDE4-inhibitor of formula III, which - in combination with standard of care treatment in PF-ILD/IPF (either nintedanib or pirfenidone)- combine both, an excellent therapeutic efficacy and an acceptable tolerability/safety profile in humans, in particular acceptable gastrointestinal adverse events/side effects.

However, only oral compositions with dosings and dose regimens that combine both, a satisfying therapeutic efficacy for the treatment of PF-ILD, preferably IPF, on the one hand and an acceptable tolerability/safety on the other hand will lead to sufficient patient compliance and thereby to a successful PF-ILD/IPF- therapy for patient.

Consequently it was the problem of the invention to provide new methods of treatment and or combination treatments for therapy of PF-ILD-patients, preferably for therapy of IPF-patients, that will combine both, an increased therapeutic efficacy (compared to standard of care treatment in PF-ILD/IPF alone, that means either nintedanib alone or pirfenidone alone) and an acceptable tolerability/safety profile, that will lead to sufficient patient compliance. With regard to the acceptable tolerability/safety profile it is of particular importance that the gastrointestinal side effects/adverse events are acceptable and that therefore the patient's compliance to the medication is achieved and maintained.

This problem of the invention was solved by the results of a clinical trial in IPF patients (phase II) over 12 weeks evaluating efficacy, safety and tolerability of the PDE4-inhibitor of formula III either alone or in combination with standard of care IPF-treatment (with either nintedanib or pirfenidone) which led to new methods of treating PF-ILD/IPF combining standard of care medication in PF-ILD/IPF (either nintedanib or pirfenidone) with 18 mg twice daily of the PDE4-inhibitor of formula III

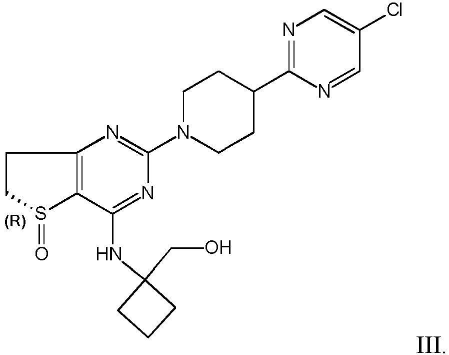

In a preferred embodiment the PDE4-inhibitor of formula III is administered to the PF-ILD patient, preferably to the IPF patient, in the dose of 18 mg twice daily.

In a first aspect, the problem of the invention was solved by a combination of the PDE4-inhibitor of formula III 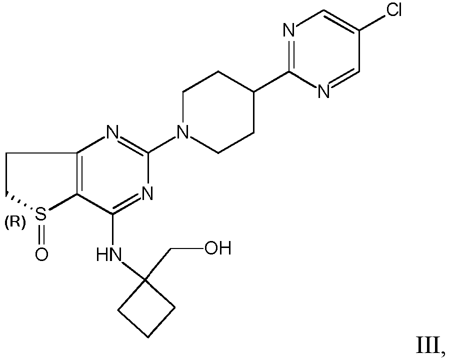 and pirfenidone. In particular, the problem of the invention was solved in this first aspect by a combination of the PDE4-inhibitor of formula III and pirfenidone, wherein the PDE4-inhibitor of formula III is administered to the patient in the dose of 18 mg per administration with two administrations per day (18 mg twice daily) and wherein pirfenidone is administered to the patient in therapeutically effective doses and dose regimens as authorized for pirfenidone (in particular doses and dose regimens which lead to a daily dose of pirfenidone between 801 mg and 2403 mg).

In a second aspect, this problem of the invention was solved by a combination of the PDE4-inhibitor of formula III 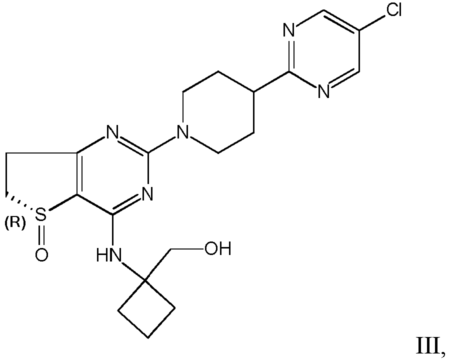 and nintedanib, wherein the PDE4-inhibitor of formula III is administered to the patient in the dose of 18 mg per administration with two administrations per day (18 mg twice daily) and wherein nintedanib is administered to the patient in therapeutically effective doses and dose regimens as authorized for nintedanib, in particular in a 150 mg dose administered twice daily or in a 100 mg dose administered twice daily.

In a preferred embodiment the PDE4-inhibitor of formula III is administered to the patient in a dose of 18 mg per administration with two administrations per day (18 mg twice daily) either in combination with pirfenidone or in combination with nintedanib.

### 2. Description of the Invention

### 2.1 Detailed Description of the Invention

In a first aspect the invention concerns an oral pharmaceutical composition comprising
- 18 mg of the PDE4B-inhibitor of formula III 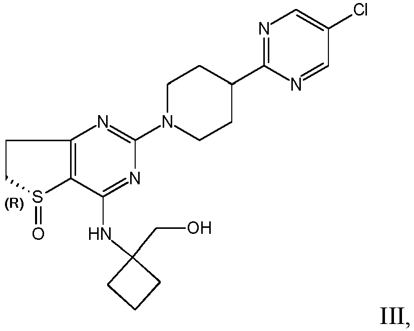
- a therapeutically effective dose of pirfenidone and
- optionally, one or more pharmaceutically acceptable carriers or excipients
   for the treatment of a patient suffering from a progressive fibrosing interstitial lung disease (PF-ILD), wherein this oral pharmaceutical composition is to be administered to said patient twice daily.

Hereby, the progressive fibrosing interstitial lung disease (PF-ILD) the patient is suffering from is preferably idiopathic pulmonary fibrosis (IPF).

Hereby, the therapeutically effective dose of pirfenidone is preferably a dose that leads to a daily dose between 801 mg and 2403 mg.

In a second aspect the invention concerns a method of treating one or more Progressive Fibrosing Interstitial Lung Diseases (PF-ILDs), comprising the step of administering orally to a patient in need thereof a therapeutically effective amount of PDE4-inhibitor of formula III 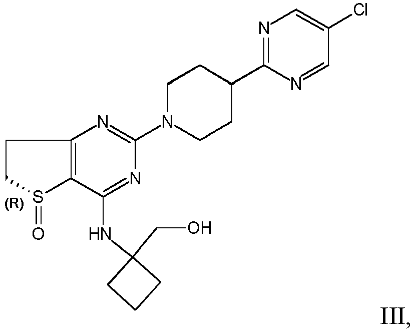 in combination with a therapeutically effective amount of pirfenidone.

In a preferred embodiment of the above-mentioned method, the PDE4-inhibitor of formula III is administered in the dose of 18 mg twice daily to the patient in need thereof in combination to a therapeutically effective amount of pirfenidone.

In a further preferred embodiment of the above-mentioned method, the Progressive Fibrosing Interstitial Lung Disease (PF-ILD) is idiopathic pulmonary fibrosis (IPF).

In a further preferred embodiment of the above-mentioned method, pirfenidone is administered in a dose and dose regimen as authorized for pirfenidone.

In a further preferred embodiment of the above-mentioned method, pirfenidone is administered in a dose and dose regimen leading to a daily dose between 801 mg and 2403 mg.

In a further preferred embodiment of the above-mentioned method, the PDE4-inhibitor of formula III in the dose of 18 mg is administered twice daily in one oral composition and the therapeutically effective amount of pirfenidone is administered in a separate oral composition.

In another preferred embodiment of the above-mentioned method, the PDE4-inhibitor of formula III in the dose of 18 mg and the therapeutically effective amount of pirfenidone is administered twice daily in the same oral composition.

The references to the methods of treatment by therapy in this description are to be interpreted as references to pharmaceutical compositions, medicaments and kits of the present invention for use in those methods.

In a third aspect the invention concerns an oral pharmaceutical composition comprising the PDE4B-inhibitor of formula III 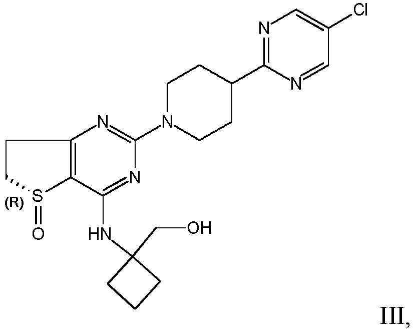 in the dose of 18 mg for use in a method of treating a patient suffering from one or more Progressive Fibrosing Interstitial Lung Diseases (PF-ILDs), wherein said oral pharmaceutical composition comprising 18 mg of the PDE4B-inhibitor of formula III is administered twice daily to the patient in need thereof in combination with a therapeutically effective amount of pirfenidone.

The therapeutically effective amount of pirfenidone is hereby preferably present in a separate oral pharmaceutical composition.

In a preferred embodiment, the above-mentioned PDE4-inhibitor of formula III is administered in a dose of 18 mg twice daily in combination with the therapeutically effective amount of pirfenidone to the patient in need thereof.

The therapeutically effective amount of pirfenidone is hereby preferably administered in a dose and dose regimen as authorized for pirfenidone, more preferably administered in a dose and dose regimen leading to a daily dose between 801 mg and 2403 mg.

In a further preferred embodiment, the above-mentioned PDE4-inhibitor of formula III is used for the treatment of idiopathic pulmonary fibrosis (IPF).

In a preferred embodiment, the 18 mg of the PDE4-inhibitor of formula III is administered twice daily in one oral composition and the therapeutically effective amount of pirfenidone is administered in a separate oral composition to the patient in need thereof.

In a preferred embodiment, the 18 mg of the PDE4-inhibitor of formula III and the therapeutically effective amount of pirfenidone is administered to the patient in need thereof twice daily in the same oral composition.

In a fourth aspect the invention concerns the use of the PDE4B-inhibitor of formula III 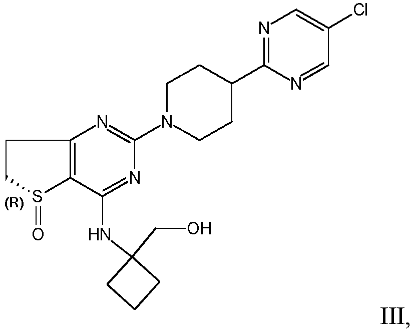 for preparing an oral pharmaceutical composition comprising a therapeutically effective amount of the PDE4B-inhibitor of formula III for use in a method of treating a patient suffering from one or more Progressive Fibrosing Interstitial Lung Diseases (PF-ILDs), wherein said oral pharmaceutical composition comprising the therapeutically effective amount of said PDE4-inhibitor of formula III is to be orally administered to said patient in combination with a therapeutically effective amount and dose regimen of pirfenidone.

In a preferred embodiment of the above-mentioned use of the PDE4B-inhibitor of formula III said oral pharmaceutical composition comprises the PDE4B-inhibitor of formula III in the dose of 18 mg and is to be administered twice daily in combination with a therapeutically effective amount of pirfenidone to the patient in need thereof.

In a further preferred embodiment of the above-mentioned use of the PDE4B-inhibitor of formula III pirfenidone is administered in a dose and dose regimen leading to a daily dose between 801 mg and 2403 mg.

In a further preferred embodiment of the above-mentioned use of the PDE4B-inhibitor of formula III, the oral pharmaceutical composition comprising 18 mg of the PDE4B-inhibitor of formula III is administered twice daily and the therapeutically effective amount of pirfenidone is administered in a separate oral composition -preferably three times daily - to the patient in need thereof.

In a preferred embodiment of the above-mentioned use of the PDE4B-inhibitor of formula III, the 18 mg of the PDE4B-inhibitor of formula III is present in the same oral pharmaceutical composition as the therapeutically effective amount of pirfenidone and is administered to the patient in need thereof twice daily.

In a ninth aspect the invention concerns a kit for the treatment of a patient suffering from a Progressive Fibrosing Interstitial Lung Disease (PF-ILD) comprising:
- a first oral pharmaceutical composition or dosage form comprising 18 mg of the PDE4B-inhibitor of formula III 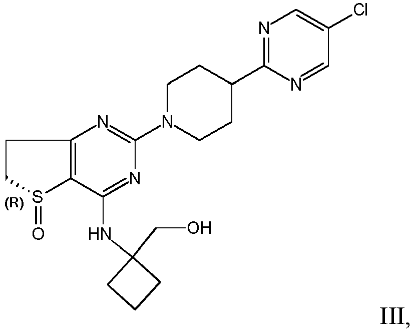 and optionally, one or more pharmaceutically acceptable carriers and/or excipients and
- a second oral pharmaceutical composition or dosage form comprising pirfenidone in a therapeutically effective dose and optionally, one or more pharmaceutically acceptable carriers and/or excipients,
wherein the first pharmaceutical composition or dosage form is to be administered twice daily.

Hereby, the Progressive Fibrosing Interstitial Lung Disease (PF-ILD) is preferably idiopathic pulmonary fibrosis (IPF).

Hereby, the first pharmaceutical composition or dosage form is to be administered simultaneously, concurrently, sequentially, successively, alternately or separately with the second pharmaceutical composition or dosage form.

Hereby, the second pharmaceutical composition or dosage form comprises pirfenidone in the dose of either 267 mg and is administered three times daily or in a dose of 534 mg and is administered three times daily or in the dose of 801 mg and is administered three times daily.

Hereby, wherein the second pharmaceutical composition or dosage form comprises pirfenidone in a daily dose between 801 mg and 2403 mg.

In an eleventh aspect, the invention concerns a therapeutic combination comprising a first oral pharmaceutical composition comprising 18 mg of the PDE4B-inhibitor of formula III 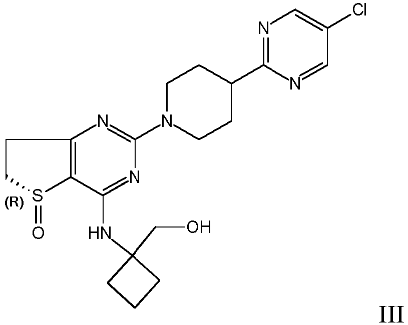 and a second oral pharmaceutical composition comprising a therapeutically effective dose of pirfenidone for use in the treatment of progressive fibrosing interstitial lung disease (PF-ILD), wherein the first oral pharmaceutical composition is administered twice daily.

In a preferred embodiment of these therapeutic combinations the progressive fibrosing interstitial lung disease (PF-ILD) is idiopathic pulmonary fibrosis (IPF).

In another preferred embodiment of these therapeutic combinations the second oral pharmaceutical composition comprises pirfenidone in a daily dose between 801 mg and 2403 mg.

In another preferred embodiment of these therapeutic combinations the second oral pharmaceutical composition comprises pirfenidone in a dose of 267 mg and is administered to the patient three times daily.

In another preferred embodiment of these therapeutic combinations the second oral pharmaceutical composition comprises pirfenidone in a dose of 534 mg and is administered to the patient three times daily.

In another preferred embodiment of these therapeutic combinations the second oral pharmaceutical composition comprises pirfenidone in a dose of 801 mg and is administered to the patient three times daily.

In a twelfth aspect the invention concerns the use of an oral pharmaceutical composition comprising 18 mg of the PDE4B-inhibitor of formula III 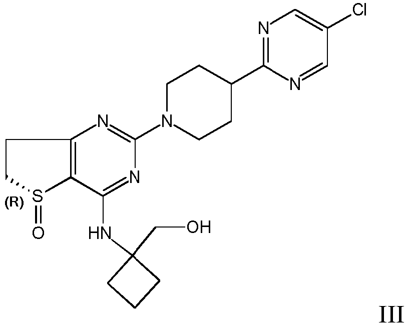 twice daily in combination with an antifibrotic background treatment of a therapeutically effective dose of pirfenidone for the treatment of patients suffering from a progressive fibrosing interstitial lung disease (PF-ILD).

In a preferred embodiment of the above-mentioned uses the progressive fibrosing interstitial lung disease (PF-ILD) is idiopathic pulmonary fibrosis (IPF).

In another preferred embodiment of the above-mentioned uses the oral pharmaceutical compositions comprising either18 mg of the PDE4B-inhibitor of formula III is a film-coated tablet.

In a thirteenth aspect the invention concerns a combination of the PDE4B-inhibitor of formula III, in particular in the dose of 18 mg administered twice daily, with a second therapeutic agent selected from the group as listed in the following table:

| **Therapeutic agent (either tradename or INN-name or company code)** | **Mechanism of Action** | **Citation** |
|---|---|---|
| Treprostinil^{™} | a synthetic analog of prostacyclin (PGI₂), vasodilator | Drug Approval Package: Remodulin (Treprostinil Sodium) NDA #021272". U.S. Food and Drug Administration (FDA). 24 December 1999. Retrieved 9 April 2020 |
| Pamrevlumab (FG-3019) | Anti- Connective tissue growth factor (CTGF) mouse antibody | World Health Organization (2015). "International Nonproprietary Names for Pharmaceutical Substances (INN). |
| | | Proposed INN: List 113" (PDF). WHO Drug Information. 29 (2) |
| PRM-151 | Recombinant pentraxin-2 recombinant human serum amyloid P/pentraxin 2 | |
| ND-L02-s0201 | anti-HSP47 si RNA lipid nanoparticle | |
| Pamufetinib (TAS-115 mesylate) | VEGFR inhibitor | |
| Dersimelagon (MT-7117) | Melanocortin 1 receptor agonist (MC1R agonist) | Suzuki et al., Skin Health Dis. 2021, e78 |
| | | https://doi.org/10.1002/ski2.78 |
| Belumosudil (KD025) | | |
| BMS-986278 | Lysophosphatidic acid receptor 1 antagonist (LPA1) | Cheng et al, J Med Chem. 2021, Nov. 11; 64 (21): 15549-15581 |
| GLPG4716 (OATD-01) | Chitinase inhibitor | Koralwski et al J Med Chem. 2020, Dec 24; 63 (24): 15527-15540 |
| Ifetroban | Thromboxane A2/prostaglandin H2 receptor antagonist | Rosenfeld et al, Cardiovascular Drug Reviews 19 (2): 97-115 |
| GB0139 | Galectin-3 Inhibitor | |
| 3-Deoxy-3-[4-(3-Fluorophenyl)-1h-1,2,3-Triazol-1-Y1]-Beta-1-Galactopyranosyl 3-Deoxy-3-[4-(3-Fluorophenyl)-1h-1,2,3-Triazol-1-Y1]-1-Thio-Beta-D-Galactopyranoside | | |
| CC-90001 | c-Jun N-terminal kinase (JNK) | |
| Ianalumab (VAY736) | Anti-B-cell activating factor (BAFF) | |
| VP01 (C21) | Angiotensin type 2 (Ang II) receptor agonist | WO2021/229244 |

In a further preferred embodiment the invention concerns a combination of the PDE4B-inhibitor of formula III, in particular in the dose of either 18 mg administered twice daily with Treprostinil^{™} as a second therapeutic agent.

In a further preferred embodiment the invention concerns a combination of the PDE4B-inhibitor of formula III, in particular in the dose of either 18 mg administered twice daily, with Pamrevlumab as a second therapeutic agent.

In a further preferred embodiment the invention concerns a combination of the PDE4B-inhibitor of formula III, in particular in the dose of either 18 mg administered twice daily, with PRM-151 as a second therapeutic agent.

In a further preferred embodiment the invention concerns a combination of the PDE4B-inhibitor of formula III, in particular in the dose of either18 mg administered twice daily with ND-L02-s0201 as a second therapeutic agent.

In a further preferred embodiment the invention concerns a combination of the PDE4B-inhibitor of formula III, in particular in the dose of either 18 mg administered twice daily, with Pamufetinib as a second therapeutic agent.

In a further preferred embodiment the invention concerns a combination of the PDE4B-inhibitor of formula III, in particular in the dose of either 18 mg administered twice daily with Dersimelagon as a second therapeutic agent.

In a further preferred embodiment the invention concerns a combination of the PDE4B-inhibitor of formula III, in particular in the dose of either 18 mg administered twice daily, with Belumosudil as a second therapeutic agent.

In a further preferred embodiment the invention concerns a combination of the PDE4B-inhibitor of formula III, in particular in the dose of either 18 mg administered twice daily, with BMS-986278 as a second therapeutic agent.

In a further preferred embodiment the invention concerns a combination of the PDE4B-inhibitor of formula III, in particular in the dose of either 18 mg administered twice daily, with GLPG4716 as a second therapeutic agent.

In a further preferred embodiment the invention concerns a combination of the PDE4B-inhibitor of formula III, in particular in the dose of either 18 mg administered twice daily, with Ifetroban as a second therapeutic agent.

In a further preferred embodiment the invention concerns a combination of the PDE4B-inhibitor of formula III, in particular in the dose of either 18 mg administered twice daily, with GB0139 as a second therapeutic agent.

In a further preferred embodiment the invention concerns a combination of the PDE4B-inhibitor of formula III, in particular in the dose of either 18 mg administered twice daily, with CC-90001 as a second therapeutic agent.

In a further preferred embodiment the invention concerns a combination of the PDE4B-inhibitor of formula III, in particular in the dose of either 18 mg administered twice daily, with Ianalumab as a second therapeutic agent.

In a further preferred embodiment the invention concerns a combination of the PDE4B-inhibitor of formula III, in particular in the dose of either 18 mg administered twice daily with VP01 as a second therapeutic agent.

In a further preferred embodiment of the above-mentioned combination, the PDE4B-inhibitor of formula III in the dose of 18 mg is administered twice daily in one oral composition and the therapeutically effective amount of one of the second therapeutic agents as listed in the above-mentioned table is administered in a separate oral composition.

In another preferred embodiment of the above-mentioned combination, the PDE4B-inhibitor of formula III in the dose of 18 mg and the therapeutically effective amount of one of the second therapeutic agents as listed in the above-mentioned table are administered together twice daily in the same oral composition.

### 2.2 Figures

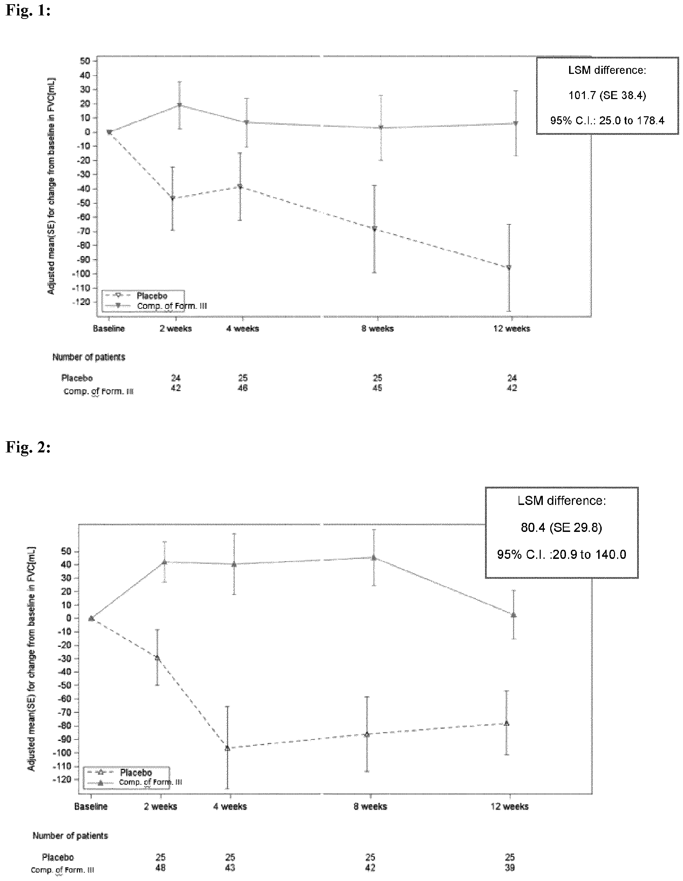

- **Fig. 1**: describes the adjusted mean (SE) for the change from baseline [ml] in Forced Vital Capacity (FVC) for the "Non-AF-group". For the "Non-AF-group" the difference of the change from baseline in FVC between the "Comp. of Form. III-group" (= "active agent group") and the "Placebo group" after 12 weeks of treatment was 101.7 ml (MMRM).
The curve with the data points depicted as solid triangles represents the group that had obtained the "Comp. of Form. III" (that means the "active agent group") and the dashed curve with the data points depicted as empty triangles represents the group that had obtained "Placebo".
- **Fig. 2**: describes the adjusted mean (SE) for the change from baseline [ml] in Forced Vital Capacity (FVC) for the complete "AF-group" ("nintedanib as antifibrotic background medication group" + "pirfenidone as antifibrotic background medication group"). For the complete "AF-group" the difference of the change from baseline in FVC between the "Comp. of Form. III-group" (= "active agent group") and the "Placebo group" after 12 weeks of treatment was 80.4 ml (MMRM).
The curve with the data points depicted as solid triangles represents the group that had obtained the "Comp. of Form. III" (that means the "active agent group") and the dashed curve with the data points depicted as empty triangles represents the group that had obtained "Placebo".

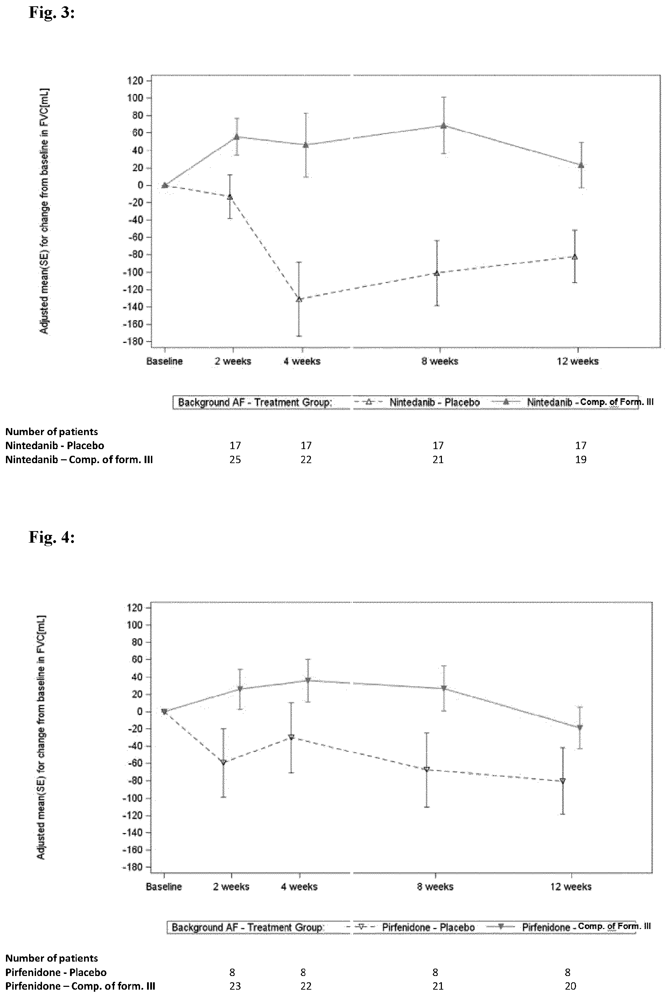

- **Fig. 3**: describes the adjusted mean (SE) for the change from baseline [ml] in Forced Vital Capacity (FVC) for that part of the "AF-group" that had obtained nintedanib as antifibrotic background medication ("nintedanib as antifibrotic background medication group"). For that part of the "AF-group" that had obtained nintedanib as antifibrotic background medication the difference of the change from baseline in FVC between the "Comp. of Form. III-group" (= "active agent group") and the "Placebo group" after 12 weeks of treatment was 105.43 ml (MMRM).
The curve with the data points depicted as solid triangles represents the group that had obtained the "Comp. of Form. III" (that means the "active agent group") and the dashed curve with the data points depicted as empty triangles represents the group that had obtained "Placebo".
- **Fig. 4**: describes the adjusted mean (SE) for the change from baseline [ml] in Forced Vital Capacity (FVC) for that part of the "AF-group" that had obtained pirfenidone as antifibrotic background medication ("pirfenidone as antifibrotic background medication group"). For that part of the "AF-group" that had obtained pirfenidone as antifibrotic background medication the difference of the change from baseline in FVC between the "Comp. of Form. III-group" (= "active agent group") and the "Placebo group" after 12 weeks of treatment was 61.30 ml (MMRM).
The curve with the data points depicted as solid triangles represents the group that had obtained the "Comp. of Form. III" (that means the "active agent group") and the dashed curve with the data points depicted as empty triangles represents the group that had obtained "Placebo".

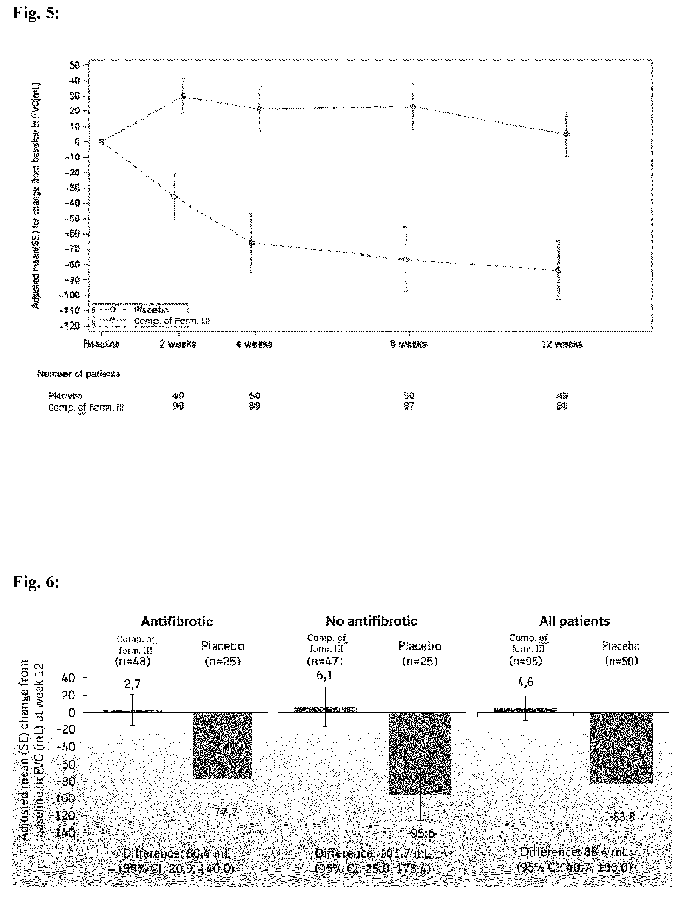

- **Fig. 5**: describes the adjusted mean (SE) for the change from baseline [ml] in Forced Vital Capacity (FVC) for the "pooled AF background" (= the "Non-AF-group" + the complete "AF-group"). For the "pooled AF background" the difference of the change from baseline in FVC between the "Comp. of Form. III-group" (= "active agent group") and the "Placebo group" after 12 weeks of treatment was 88.38 ml (MMRM).
The curve with the data points depicted as solid circles represents the group that had obtained the "Comp. of Form. III" (that means the "active agent group") and the dashed curve with the data points depicted as empty circles represents the group that had obtained "Placebo".
- **Fig. 6**: describes the adjusted mean (SE) change from baseline in Forced Vital Capacity (FVC) [ml] at week 12 (MMRM) for the complete "AF-group" (= "antifibrotic"), for the "Non-AF-group" (="No antifibrotic") and for the "pooled AF background" (="All patients").

### 3. Clinical Trial

### 3.1 Detailed Description of the Clinical Trial

The study was open to adults with idiopathic pulmonary fibrosis (IPF) who were at the time of recruitment at least 40 years old. People taking standard medicines for IPF, including antifibrotic medicines, were allowed to continue taking them throughout the study.

The purpose of the study was to find out whether the PDE4 inhibitor of formula III 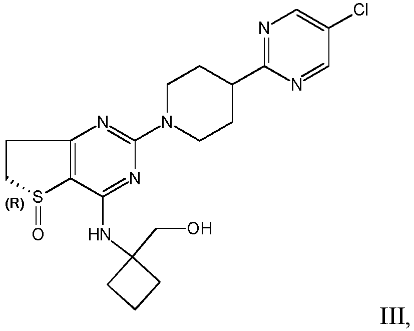 could slow down the worsening of lung function. Participants were in the study for about 4 months. During this time, they visited the study site about 7 times. At the beginning, they visited the study site every 2 weeks. After 1 month of treatment, they visited the study site every 4 weeks.

The participants were divided into 2 groups by chance (the "active agent group" and the "placebo group"). One tablet comprising 12 mg of the compound of formula III and one tablet comprising 6 mg of the compound of formula III (,,the active agent tablets" resulting in a 18 mg single dose, see Table 1) was administered orally to the patients of the first group twice daily (b.i.d.). One 12 mg placebo tablet and one 6 mg placebo tablet (both containing no active ingredient, see Table 1) was administered orally to the patients of the second group twice daily (b.i.d.). The placebo tablets looked like the tablets of the active ingredient (=the compound of formula III).

**Table 1: Composition of the "active agent tablets" and of the "placebo tablets" as administered in the clinical trial (one 12 mg and one 6 mg tablet of the "active agent tablets" or "placebo tablets" per administration, twice administrations per day)**

| **Ingredients** | **Placebo 6 mg tablet (mg/tablet)** | **Placebo 12 mg tablet (mg/tablet)** | **Comp. of form. III 6 mg tablet (mg/tablet)** | **Comp. of form. III 12 mg tablet (mg/tablet)** | **Function** | **Reference to Standards** |
|---|---|---|---|---|---|---|
| Comp. of form. III | - | - | 6.00 | 12.00 | Active Ingredient | Company Standard |
| Mannitol | 103.53 | 216.58 | | | | |
| Lactose monohydrate | - | - | 138.72 | 277.44 | Filler | Ph. Eur., NF |
| Cellulose, microcrystalline | 67.425 | 141.05 | 50.25 | 100.50 | Filler | Ph. Eur., NF |
| Croscarmellose sodium | - | - | 4.02 | 8.04 | Disintegrant | Ph. Eur., NF |
| Magnesium stearate | 3.045 | 6.37 | 2.01 | 4.02 | Lubricant | Ph. Eur., NF |
| **Sub-total (tablet core)** | **174.00** | **364.00** | **201.00** | **402.00** | | |
| Film-coating mixture red | 5.00 | 10.00 | 5.00 | 10.00 | Coating agent | |
| Water, purified (removed during processing) | q.s. | q.s. | q.s. | q.s. | Solvent | Ph. Eur., USP |
| **Total (film-coated tablet)** | **179.00** | **374.00** | **206.00** | **412.00** | | |

The "active agent group" and the "placebo group" were further distributed to two different groups regarding background medication: the "AF-group" ("antifibrotic background medication group") and the "Non-AF-group" ("no antifibrotic background medication group"). The patients that were part of the "Non-AF group" did not obtain an antifibrotic background medication of either nintedanib or pirfenidone (in doses and dose regimens as authorized) during the trial (that means either the PDE4B inhibitor of formula III in the 18 mg dose b.i.d (=twice daily) or placebo b.i.d (=twice daily) was administered alone) and the patients that were part of the "AF-group" obtained an antifibrotic background medication of either nintedanib or pirfenidone during the trial (that means either the PDE4B inhibitor of formula III in the 18 mg dose b.i.d (=twice daily) or placebo b.i.d (=twice daily) was administered in combination with either nintedanib or with pirfenidone. The combination of both nintedanib and pirfenidone as background medication was not allowed in the "AF-group".

The participants performed lung function tests at study visits ("Forced Vital Capacity (FVC)"). The results of the lung changes in function tests were compared between the "active agent group" and the "placebo group" (both within the "AF-group and within the "Non AF-group"). The doctors also regularly checked the general health of the participants.

For the study 147 IPF patients with the following inclusion criteria have been recruited:
- Patients aged ≥40 years when signing the informed consent.
- Diagnosis: IPF based on 2018 ATS/ERS/JRS/ALAT Guideline as confirmed by the investigator based on chest High Resolution Computed Tomography Scan (HRCT) scan taken within 12 months of Visit 1 and if available surgical lung biopsy.
   and
   Usual interstitial pneumonia (UIP) or probable UIP HRCT pattern consistent with the clinical diagnosis of IPF, as confirmed by central review prior to Visit 2*,
   if indeterminate HRCT finding IPF may be confirmed locally by (historical) biopsy
- Stable for at least 8 weeks prior to Visit 1.
- Patients have to be either :
   - not on therapy with nintedanib or pirfenidone for at least 8 weeks prior to Visit 1 (combination of nintedanib plus pirfenidone not allowed)
      or
   - on stable* therapy with nintedanib or pirfenidone for at least 8 weeks prior to Visit 1 and planning to stay stable on this background therapy after randomisation
   [*stable therapy is defined as the individually and general tolerated regimen of either pirfenidone or nintedanib, (combination of nintedanib plus pirfenidone not allowed)]
- Forced Vital Capacity (FVC) ≥45% of predicted normal at Visit 1
- Diffusion capacity of the lung for carbon monoxide (DLCO) (corrected for haemoglobin [Hb] [Visit 1]) ≥ 25% to < 80% of predicted normal at Visit 1.
- Signed and dated written informed consent in accordance with ICH-GCP and local legislation prior to admission to the trial.

For the participating IPF patients the following exclusion criteria were applied:
- Relevant airways obstruction (pre-bronchodilator Forced Expiratory Volume in one second (FEV1)/Forced Vital Capacity (FVC) < 0.7) at Visit 1.
- In the opinion of the Investigator, other clinically significant pulmonary abnormalities.
- Acute IPF exacerbation within 4 months prior to screening and/or during the screening period (investigator-determined).
- Lower respiratory tract infection requiring antibiotics within 4 weeks prior to Visit 1 and/or during the screening period.
- Major surgery (major according to the investigator's assessment) performed within 3 months prior to Visit 1 or planned during the course of the trial. (Being on a transplant list is allowed).
- Any documented active or suspected malignancy or history of malignancy within 5 years prior to Visit 1, except appropriately treated basal cell carcinoma of the skin, "under surveillance" prostate cancer or in situ carcinoma of uterine cervix.
- Evidence of active infection (chronic or acute) based on clinical exam or laboratory findings at Visit 1 or at Visit 2.
- Any suicidal behaviour in the past 2 years (i.e. actual attempt, interrupted attempt, aborted attempt, or preparatory acts or behavior).
- The patient has a confirmed infection with SARS-CoV-2 within the 4 weeks prior to Visit 1 and/or during the screening period.
- Further exclusion criteria apply.

The trial was a randomised, double-blind, placebo-controlled parallel group study. During the 12 weeks of the study the lung function of all participating IPF patients has been monitored by measuring the change from baseline in Forced Vital Capacity (FVC) ("primary outcome measure"). During the 12 weeks of the treatment period and one week after the treatment period (time frame: up to 13 weeks) the Treatment Emergent Adverse Events (TEAE) have been monitored for all participating IPF patients ("secondary outcome measures").

### 3.2 Results of the Phase II clinical trial:

### 3.2.1 Overall Population

Out of the 147 patients, all who prematurely discontinued from the trial medication were from the "active agent group" for both, the "AF-group" 10/49 (20.4%) and the "Non-AF-group" 5/49 (10.4%).

The most frequent reason for premature discontinuation was an adverse event which accounts for 100% of the discontinuations in the "AF-group" 10/10 and for 60% of the discontinuations in the "Non-AF-group" 3/5 (see Table 2).

**Table 2: Overview of the clinical trial**

| | AF-group | | | | | | Non-AF-group | | | | | |
|---|---|---|---|---|---|---|---|---|---|---|---|---|
| | Placebo | | Comp. of form. III | | Total | | Placebo | | Comp. of form. III | | Total | |
| | N | % | N | % | N | % | N | % | N | % | N | % |
| Screened | | | | | 98 | | | | | | 135 | |
| Not randomized | | | | | 24 | | | | | | 62 | |
| Randomized | | | | | 74 | | | | | | 73 | |
| Not treated | | | | | 0 | | | | | | 0 | |
| Treated | 25 | 100 | 49 | 100 | **74** | 100 | 25 | 100 | 48 | 100 | **73** | 100 |
| Completed planned observation period | 25 | 100 | 42 | 85.7 | 67 | 90.5 | 25 | 100 | 44 | 91.7 | 69 | 94.5 |
| Not completed planned observation period | 0 | 0 | 7 | 14.3 | 7 | 9.5 | 0 | 0 | 4 | 8.3 | 4 | 5.5 |
| Lost to follow-up | 0 | 0 | 0 | 0 | 0 | 0 | 0 | 0 | 0 | 0 | 0 | 0 |
| Withdrawal by patient | 0 | 0 | 3 | 6.1 | 3 | 4.1 | 0 | 0 | 2 | 4.2 | 2 | 2.7 |
| Death | 0 | 0 | 1 | 2.0 | 1 | 1.4 | 0 | 0 | 1 | 2.1 | 1 | 1.4 |
| Other | 0 | 0 | 3 | 6.1 | 3 | 4.1 | 0 | 0 | 1 | 2.1 | 1 | 1.4 |
| Completed planned treatment period | 25 | 100 | 39 | 79.6 | 64 | 86.5 | 25 | 100 | 43 | 89.6 | 68 | 93.2 |
| Not completed planned treatment period | 0 | 0 | 10 | 20.4 | **10** | 13.5 | 0 | 0 | 5 | 10.4 | **5** | 6.8 |
| Adverse Event | 0 | 0 | 10 | 20.4 | **10** | 13.5 | 0 | 0 | 3 | 6.3 | **3** | 4.1 |
| Protocol deviation | 0 | 0 | 0 | 0 | 0 | 0 | 0 | 0 | 0 | 0 | 0 | 0 |
| Lost to follow-up | 0 | 0 | 0 | 0 | 0 | 0 | 0 | 0 | 0 | 0 | 0 | 0 |
| Withdrawal by Patient | 0 | 0 | 0 | 0 | 0 | 0 | 0 | 0 | 1 | 2.1 | 1 | 1.4 |
| Other | 0 | 0 | 0 | 0 | 0 | 0 | 0 | 0 | 1 | 2.1 | 1 | 1.4 |

Table 3 shows that the time since diagnosis of IPF was longer in the "AF-group" (4.33 yrs.) vs. in the "Non-AF-group" (2.54 yrs.) and was balanced between placebo control and active agent arms.

**Table 3: Demographic Data for treated patients: Time since diagnosis**

| | AF-group | | | | | | Non-AF-group | | | | | |
|---|---|---|---|---|---|---|---|---|---|---|---|---|
| | Placebo | | Comp. of form. III | | Total | | Placebo | | Comp. of form. III | | Total | |
| | N | % | N | % | N | % | N | % | N | % | N | % |
| Time since first diagnosis (years) | | | | | | | | | | | | |
| N | 25 | | 49 | | 74 | | 25 | | 48 | | 73 | |
| Mean (years) | 3.9 | | 4.55 | | 4.33 | | 2.24 | | 2.70 | | 2.54 | |
| Time since diagnosis (years) | | | | | | | | | | | | |
| < 1 year | 4 | 16.0 | 8 | 16.3 | 12 | 16.2 | 8 | 32.0 | 14 | 29.2 | 22 | 30.1 |
| 1 to <3 years | 8 | 32.0 | 14 | 28.6 | 22 | 29.7 | 11 | 44.0 | 18 | 37.5 | 29 | 39.7 |
| 3 to 5 years | 5 | 20.0 | 9 | 18.4 | 14 | 18.9 | 4 | 16.0 | 6 | 12.5 | 10 | 13.7 |
| >5 years | 8 | 32.0 | 18 | 36.7 | 26 | 35.1 | 2 | 8.0 | 10 | 20.8 | 12 | 16.4 |

Table 4 shows that the IPF patients in the "AF-group" used more nintedanib as background medication than pirfenidone (in the "active agent group" 53.1 % nintedanib and 46.9% pirfenidone, in the "placebo group" 68 % nintedanib and 32 % pirfenidone). Furthermore, Table 4 shows that for the IPF-patients from the "AF-group" the passed time since IPF-diagnosis was longer (4.6 years for the "active agent group" and 3.9 years for the "placebo group") than for the patients of the "Non-AF-group" (2.7 years for the "active agent group" and 2.2 years for the "placebo group").

**Table 4: Characterization of the patients as treated in the different groups**

| | **AF-group** | | **Non-AF-group** | | **All patients** | |
|---|---|---|---|---|---|---|
| | **Comp. of form. III (n=49)** | **Placebo (n=25)** | **Comp. of form. III (n=48)** | **Placebo (n=25)** | **Comp. of form. III (n=97)** | **Placebo (n=50)** |
| Time since diagnosis, years, mean (SD) | 4.6 (3.7) | 3.9 (3.3) | 2.7 (2.4) | 2.2 (2.6) | 3.6 (3.2) | 3.1 (3.0) |
| Antifibrotic background medication, n (%) | | | | | | |
| nintedanib | 26 (53.1) | 17 (68.0) | 0 | 0 | 26 (26.8) | 17 (34.0) |
| pirfenidone | 23 (46.9) | 8 (32.0) | 0 | 0 | 23 (23.7) | 8 (16.0) |
| FVC, ml, mean (SD) | 2876 (753) | 2690 (890) | 2783 (835) | 2865 (1015) | 2830 (792) | 2777 (949) |
| FVC, % predicted, mean (SD) | 75.8 (17.9) | 71.7 (12.3) | 80.4 (16.0) | 82.1 (17.7) | 78.1 (17.1) | 76.9 (16.0) |
| Dlco, % predicted, mean (SD) | 49.0 (18.3) | 46.5 (16.2) | 52.0 (16.7) | 48.3 (12.1) | 50.5 (17.5) | 47.4 (14.2) |

### 3.2.2 Results regarding Efficacy of the PDE4-inhibitor of formula III

### 3.2.2.1 Results for "Non-AF-group"

### Adjusted means estimate (Mixed Model Repeated Measures, MMRM):

The IPF patients of the "Non AF-group" obtained either tablets containing 18 mg of the compound of formula III twice daily (="active agent group") or placebo tablets without active ingredient twice daily (hereby the "Placebo-tablets" were with respect to look, smell, taste etc. not distinguishable from the "active agent tablets" containing 18 mg of the compound of formula III). The patients of the "Non-AF-group" obtained no standard of care background medication for the treatment of IPF during the study period of 12 weeks (that means no nintedanib background medication and no pirfenidone background medication). Each tested patient performed lung function tests in regular intervals at each study visit. From the results of these lung function tests the change from baseline in FVC (in ml) was determined for the different time points in the study (see Figure 1). The patient data from the lung function tests at the different time points within the study period of 12 weeks was analyzed by Mixed Model Repeated Measures (MMRM). MMRM is an established method of analyzing longitudinal data allowing to use a set of repeated measurements of a patient in the estimation process (Fitzmaurice et al., "Longitudinal Data Analysis, Chapman & Hall/CRC, New York (2009)).

The change from baseline in FVC from the beginning of the study to week 12 was - 95.62 ml for the "Placebo group" and + 6.10 ml for the "Active agent group" resulting in a difference in the change from baseline in FVC from the beginning of the study to week 12 between "active agent group" and "Placebo group" of 101.72 ml (see Table 5, Figure 1 and Figure 6). Whereas the change from baseline in FVC substantially decreased in the "Placebo group" during the 12 weeks long study period (which means that the IPF disease progressed substantially in these untreated patients), the change from baseline in FVC in the "active agent group" (that had obtained the PDE4-inhibitor of formula III in the 18 mg dose twice daily) stayed stable or was even slightly increased during the same 12 weeks long study period (see Fig. 1 and Fig. 6). Consequently the PDE4-inhibitor of formula III in the 18 mg dose twice daily shows an impressive therapeutic efficacy in IPF-patients over the treatment period of 12 weeks without standard of care background medication of either nintedanib or pirfenidone.

**Table 5: Adjusted mean (SE) for change in baseline in FVC (ml) at 12 weeks (MMRM) for "non-AF-group"**

| | Baseline (1) | | | Change from baseline to week 12 | | | | Comparison vs. Placebo | | | |
|---|---|---|---|---|---|---|---|---|---|---|---|
| | | | | | | 95% confidence interval | | | | 95% confidence interval | |
| | N | Mean | SD | Adjusted Mean [2] | SE | lower | upper | Adjusted Mean [2] | SE | lower | upper |
| FVC [ml] | | | | | | | | | | | |
| Placebo | 25 | 2864.92 | 1015.10 | **-95.62** | 30.75 | -157.13 | -34.10 | | | | |
| Comp. of Form. III | 47 | 2766.51 | 836.26 | **6.10** | 22.90 | -39.67 | 51.88 | **101.72** | 38.35 | 25.02 | 178.41 |

### 3.2.2.2 Results for "AF-group"

### Adjusted means estimate (MMRM):

The IPF patients of the "AF-group" obtained either tablets containing 18 mg of the compound of formula III twice daily (="active agent group") or similar looking placebo tablets without active ingredient twice daily (= "Placebo group"). Additionally, the patients of the "AF-group" obtained standard of care background medication for the treatment of IPF during the study period of 12 weeks (that means either nintedanib background medication at doses/dose regimen as authorized or pirfenidone background medication at doses/dose regimen as authorized, the combination of both nintedanib and pirfenidone as background medication was not allowed). Each tested patient performed lung function tests in regular intervals at each study visit. From the results of these lung function tests the change from baseline in FVC (in ml) was determined for the different time points in the study (see Figure 2). The patient data from the lung function tests at the different time points within the study period of 12 weeks was analyzed by Mixed Model Repeated Measures (MMRM).

The change from baseline in FVC from the beginning of the study to week 12 was - 77.7 ml for the "Placebo group" and + 2.72 ml for the "Active agent group" (see Table 6, Figure 2 and Figure 6) resulting in a difference in the change from baseline in FVC from the beginning of the study to week 12 between "active agent group" and "Placebo group" of 80.42 ml. Whereas the change from baseline in FVC decreased in the "Placebo group" (the "Placebo group" of the "AF-group" obtained antifibrotic background medication only, that means nintedanib only or pirfenidone only) during the 12 weeks long study period (which means that the IPF disease progressed in these patients on antifibrotic background medication alone), the change from baseline in FVC in the "active agent group" (that had obtained the PDE4-inhibitor of formula III in the 18 mg dose twice daily in combination with antifibrotic background medication of either nintedanib or pirfenidone) stayed stable or was even slightly increased during the same 12 weeks long study period (see Fig. 2 and Fig. 6). Consequently the PDE4-inhibitor of formula III in the 18 mg dose twice daily shows in combination with antifibrotic background medication (either nintedanib or pirfenidone) an impressively improved therapeutic efficacy in IPF-patients over the treatment period of 12 weeks compared to the therapeutic effect in IPF-patients that had been treated by the same antifibrotic background medication alone.

This change from baseline in FVC to week 12 compared between "active agent group" and "Placebo group" of 80.42 ml for the "AF-group" is smaller than the 101.72 ml for the "Non AF-group". However, this is understandable, since in the "AF-group" also the antifibrotic background medication with nintedanib or pirfenidone should be responsible for a certain therapeutic base effect.

**Table 6: Adjusted mean (SE) for change in baseline in FVC (ml) at week 12 (MMRM) for "AF-group"**

| | Baseline (1) | | | Change from baseline to week 12 | | | | Comparison vs. Placebo | | | |
|---|---|---|---|---|---|---|---|---|---|---|---|
| | | | | | | 95% confidence interval | | | | 95% confidence interval | |
| | N | Mean | SD | Adjusted Mean [2] | SE | lower | upper | Adjusted Mean [2] | SE | lower | upper |
| FVC [ml] | | | | | | | | | | | |
| For Placebo | 25 | 2690.00 | 889.99 | **-77.70** | 23.60 | -124.87 | -30.53 | | | | |
| For the Comp. of Form. III | 48 | 2865.29 | 757.31 | **2.72** | 18.13 | -33.46 | 38.89 | **80.42** | 29.82 | 20.85 | 139.99 |

However, when the results from the "AF-group" were analyzed separately by the type of antifibrotic background medication that was administered to the patient (that means separation of the "nintedanib background medication group" from the "pirfenidone background medication group" within the "AF-group"), a trend to a higher therapeutic efficacy in combination with 18 mg of the PDE4-inhibitor of formula III administered twice daily was observed for the group that had obtained nintedanib as background medication compared to the group that had obtained pirfenidone as background medication.

This trend can be concluded from the data as summarized in Table 7: here for the group that had obtained nintedanib as background medication an adjusted mean difference between changes from baseline to week 12 between the "active agent group" and the "Placebo group" of **105.43 ml** was calculated from the trial data, whereas for the group that had obtained pirfenidone as background medication an adjusted mean difference between changes from baseline to week 12 between the "active agent group" and the "Placebo group" of only **61.30 ml** was calculated from the trial data (see Table 7).

In case you do not differentiate between nintedanib or pirfenidone as background medication an adjusted mean difference between changes from baseline to week 12 between the "active agent group" and the "Placebo group" of **80.42 ml** was determined (see Table 6 in comparison) - a value that lies more or less in between.

The course of development for the change from baseline in FVC over the 12 weeks of treatment can be derived from Fig. 3 for the "nintedanib as background medication group" and from Fig. 4 for the "pirfenidone as background medication group".

**Table 7: Adjusted mean (SE) for change in baseline in FVC (ml) at 12 weeks (MMRM) for "AF-group"/differentiation between nintedanib background medication and pirfenidone background medication**

| | | **Baseline** | | **Week 12** | | **Change from Baseline to Week 12** | | | | **Comparison vs. Placebo** | | | |
|---|---|---|---|---|---|---|---|---|---|---|---|---|---|
| | | | | | | | | 95 % Confidence Interval | | | | 95 % Confidence Interval | |
| | N | Mean FVC (ml) | SD | Mean FVC (ml) | SD | Adjust. mean | SE | lower | upper | Adjust. mean difference | SE | lower | upper |
| **nintedanib** | | | | | | | | | | | | | |
| Placebo | 17 | 2488.71 | 825.70 | 2418.47 | 803.13 | **-82.01** | 30.03 | -142.81 | -21.22 | **105.43** | 40.33 | 23.88 | 186.97 |
| Comp. of form. III | 25 | 2881.36 | 728.54 | 2787.37 | 716.21 | **23.41** | 26.17 | -29.42 | 76.24 | | | | |

| **pirfenidone** | | | | | | | | | | | | | |
|---|---|---|---|---|---|---|---|---|---|---|---|---|---|
| Placebo | 8 | 3117.75 | 921.85 | 3034.88 | 924.25 | **-80.06** | 38.59 | -160.42 | 0.30 | **61.30** | 45.49 | -33.38 | 155.99 |
| Comp.of form. III | 23 | 2847.83 | 803.50 | 2879.35 | 756.15 | **-18.76** | 23.86 | -68.29 | 30.77 | | | | |

Consequently the data from the clinical trial indicate that the combination of 18 mg of the PDE4-inhibitor of formula III administered twice daily and of nintedanib (in doses and dose regimens as authorized) might have even a better therapeutic efficacy than the combination of 18 mg of the PDE4-inhibitor of formula III administered twice daily and of pirfenidone (in doses and dose regimens as authorized).

### 3.2.2.3 Results for "pooled background therapy" ("Non-AF-group" + "AF-group")

### Adjusted means estimate (MMRM):

The combined results for the "Non AF-group" and the "AF-group" led to the results as shown in Figure 5 or Fig. 6 or as summarized in Table 8.

Whereas the change from baseline in FVC steadily decreased in the "Placebo group" (that had obtained either antifibrotic background medication or not, depending whether the patient was in the "Non-AF-group" or in the "AF-group") during the 12 weeks long study period, the change from baseline in FVC in the "active agent group" (that had obtained the PDE4-inhibitor of formula III in the 18 mg dose twice daily either alone or in combination with antifibrotic background medication of either nintedanib or pirfenidone, depending on whether the patient was in the "Non AF-group" or in the "AF-group") stayed stable or was even slightly increased during the same 12 weeks long study period (see Fig. 5 and Fig. 6).

The change from baseline in FVC to week 12 was for the "active agent group" -4.59 ml and for the "Placebo group" -83.79 ml, if you consider all results (that means the results for "Non-AF-group" + "AF-group"). The Change from baseline in FVC to week 12 compared between "active agent group" and "Placebo group" was calculated to be 88.38 ml, if you consider all results (that means the results for "Non-AF-group" + "AF-group").

**Table 8: Adjusted mean (SE) for change in baseline in FVC (ml) at week 12 (MMRM) for "pooled background therapy" (="Non-AF-group" + "AF-group")**

| | Baseline (1) | | | Change from baseline to week 12 | | | | Comparison vs. Placebo | | | |
|---|---|---|---|---|---|---|---|---|---|---|---|
| | | | | | | 95% confidence interval | | | | 95% confidence interval | |
| | N | Mean | SD | Adjusted Mean [2] | SE | lower | upper | Adjusted Mean [2] | SE | lower | upper |
| FVC [ml] | | | | | | | | | | | |
| Placebo | 50 | 2777.46 | 948.93 | **-83.79** | 19.20 | -121.78 | -45.80 | | | | |
| Comp. of Form. III | 95 | 2816.42 | 794.64 | **4.59** | 14.53 | -24.14 | 33.32 | **88.38** | 24.08 | 40.74 | 136.02 |

### 3.2.3 Results regarding Safety/Tolerability

### 3.2.3.1 Results regarding Safety/Tolerability of the PDE4-inhibitor of formula III within all treatments arms ("Non-AF-group" and "AF-group")

In the "active agent group" 67 % of the patients experienced an adverse event (AE): 64.6 % in the "Non-AF-group" and 73.5% in the "AF-group". In the "Placebo group" 60% of the patients experienced an AE: 52 % in the "Non-AF-group" and 68 % in the "AF-group" (see Table 9). Within all tested groups only 8.8 % of the patients experienced an AE leading to the discontinuation of the trial drug, 7.5 % of the patients experienced a serious adverse event (see Table 10) and 2 patients (that means 1.4 %) of the patients experienced an AE resulting in death (both in the "active agent group", one due to a Covid-19 pneumonitis, one due to an unconfirmed case of vasculitis).

**Table 9: Overall Summary of Adverse Events during the clinical trial**

| | **Placebo** | | | **Compound of formula III** | | | **Total** |
|---|---|---|---|---|---|---|---|
| | **Non-AF** | **AF** | **Total** | **Non-AF** | **AF** | **Total** | **N=147, n(%)** |
| **Number of patients with** | **N=25, n(%)** | **N=25, n(%)** | **N=50, n(%)** | **N=48, n(%)** | **N=49, n(%)** | **N=97, n(%)** | |
| Any AE | 13 (52.0) | 17 (68.0) | 30 (60.0) | 31 (64.6) | 36 (73.5) | 67 (69.1) | 97 (66.0) |
| AEs leading to discontinuation of trial drug | - | - | - | 3 (6.3) | 10 (20.4) | 13 (13.4) | 13 ( 8.8) |
| Severe AEs | 1 (4.0) | 1 (4.0) | 2 (4.0) | 2 (4.2) | 2 (4.1) | 4 (4.1) | 6 (4.1) |
| Serious AEs | 5 (20.0) | - | 5 (10.0) | 3 (6.3) | 3 (6.1) | 6 (6.2) | 11 (7.5) |
| AE resulting in death | - | - | - | 1 (2.1) | 1 (2.0) | 2 (2.1) | 2 ( 1.4) |

**Table 10: Overall Summary of serious adverse events during clinical trial**

| | **Placebo** | | | **Compound of formula III** | | |
|---|---|---|---|---|---|---|
| | **Non-AF** | **AF** | **Total** | **Non-AF** | **AF** | **Total** |
| **Number of patients with** | **N=25, n(%)** | **N=25, n(%)** | **N=50, n(%)** | **N=48, n(%)** | **N=49, n(%)** | **N=97, n(%)** |
| Patients with ≥ 1 SAE | 5 (20.0) | - | 5 (10.0) | 3 (6.3) | 3 (6.1) | 6 (6.2) |
| **General disorders and administration side conditions** | - | - | - | - | 2 (4.1) **[1 (2.1)]*** | 2 (2.1) |
| Condition aggravated | - | - | - | - | 2 (4.1) | 2 (2.1) |
| **Infections and Infestations** | 2 (8.0) | - | 2 (4.0) | 1 (2.1) | - | 1 (1.0) |
| Covid-19 pneumonia | - | - | - | **1** (2.1)* | - | 1 (1.0) |
| Urosepsis | 1 (4.0) | - | 1 (2.0) | - | - | - |
| Wound infection | 1 (4.0) | - | 1 (2.0) | - | - | - |
| **Respiratory, thoracic and mediastinal disorders** | - | - | - | 1 (2.1) | - | 1 (1.0) |
| COPD | - | - | - | 1 (2.1) | - | 1 (1.0) |
| Dyspnoea | - | - | - | 1 (2.1) | - | 1 (1.0) |
| **Nervous system disorders** | 1 (4.0) | - | 1 (2.0) | - | 1 (2.0) | 1 (1.0) |
| Peripheral nerve paresis | - | - | - | - | 1 (2.0) | 1 (1.0) |
| Transient ischaemic attack | 1 (4.0) | - | 1 (2.0) | - | - | - |
| **Cardiac disorders** | - | - | - | 1 (2.1) | - | 1 (1.0) |
| Cardiac failure congestive | - | - | - | 1 (2.1) | - | 1 (1.0) |
| **Metabolism and nutrition disorders** | 1 (4.0) | - | 1 (2.0) | - | - | - |
| Diabetic metabolic decompensation | 1 (4.0) | - | 1 (2.0) | - | - | - |
| **Neoplasms benign, malignant and unspecified** | - | - | - | 1 (2.1) | - | 1 (1.0) |
| Lung neoplasm | - | - | - | 1 (2.1) | - | 1 (1.0) |
| **Skin and subcutaneous tissue disorders** | 1 (4.0) | - | 1 (2.0) | - | - | - |
| Dermatitis exfoliative | 1 (4.0) | - | 1 (2.0) | - | - | - |
| **Vascular disorders** | - | - | - | - | 1 (2.0) | 1 (1.0) |
| Vasculitis | - | - | - | - | **1 (2.0)*** | 1 (1.0) |

| | | | | | | |
|---|---|---|---|---|---|---|
| * Fatal events in bold | | | | | | |

The most frequently reported adverse events (AE) were gastrointestinal disorders, of which diarrhoea was the most frequent AE (in more than 10 % of all patients diarrhoea was observed (see Table 11).

Within both groups, the "AF-group" and the "Non-AF-group", for the corresponding "active agent groups" (wherein the compound of formula III was administered either in combination with antifibrotic background medication or alone) an increased frequency of the adverse event diarrhoea compared to the respective "Placebo groups" was observed. Three patients from the "active agent group" discontinued treatment due to AE diarrhoea, all male of ≥ 65 years of age from the "AF-group" under nintedanib background treatment.

Within in the "Non-AF-group", diarrhoea was reported approximately twice as frequently for the "active agent group" (16.7 %) than for the corresponding "Placebo group" (8 %) (see Table 11). Within the "AF-group", diarrhoea was also reported approximately twice as frequently for the "active agent group" (30.6 %) than for the corresponding "Placebo group" (16.0 %) (see Table 11).

However, the overall level of reported diarrhoea within the "AF-group" was also increased compared to the "Non-AF-group" (see Table 11, within the "Placebo-group" 16% of diarrhoea in the "AF-group" compared to 8% diarrhoea in the "Non-AF-group"; within the "Comp. of Form. III-group" 30.6% of diarrhoea in the "AF-group" compared to 16.7% diarrhoea in the "Non-AF-group").

Consequently, the PDE4-inhibitor of formula III administered bid in a dose of 18 mg seems to lead to an increased frequency of the AE diarrhoea, but also the antifibrotic background medication, in particular background medication with nintedanib, seems to contribute significantly to the increased frequency of reported cases of diarrhoea in the "AF-group" (compared to the "Non-AF-group").

Nevertheless, all cases of reported diarrhoea within the "AF-group" and within the "Non-AF-group" were non-serious adverse events (see Table 10).

The majority of reported diarrhoea events were mild with the exception of two moderate cases of diarrhoea (2.1 %) and one severe case of diarrhoea (1.0 %), which all occured in males and in the "active agent group" of the "AF-group" under nintedanib background therapy. Additionally one severe case of diarrhoea occured in a 64-year-old female in the "Placebo-group" of the "AF-group" under nintedanib background therapy.

Overall, the most frequently reported AE leading to discontinuation was diarrhoea (n=4), whereby all these cases were part of the "AF-group" and were on nintedanib background medication.

In none of the cases of reported diarrhoea within all treatment arms a case of dehydration associated with diarrhoea had been observed.

In all treatment arms, no cases of hypokalaemia had been reported.

Apart from gastrointestinal disorders (in particular diarrhoea) only single adverse events have been reported over all treatment arms (in the "AF-group" and in the "Non-AF-group", in the "active agent groups" and in the "Placebo-groups") without any specific pattern (see Table 11).

**Table 11: Frequently reported Adverse Events (AEs) during the clinical trial**

| | **Placebo** | | | **Compound of Formula III** | | |
|---|---|---|---|---|---|---|
| | **Non-AF** | **AF** | **Total** | **Non-AF** | **AF** | **Total** |
| | **N=25, n(%)** | **N=25, n(%)** | **N=50, n(%)** | **N=48, n(%)** | **N=49, n(%)** | **N=97, n(%)** |
| **Gastrointestinal disorders** | 4 (16.0) | 8 (32.0) | 12 (24.0) | 13 (27.1) | 18 (36.7) | 31 (32.0) |
| Diarrhoea | **2 (8.0)** | **4 (16.0)** | 6 (12.0) | **8 (16.7)** | **15 (30.6)** | 23 (23.7) |
| Flatulence | 1 (4.0) | 1 (4.0) | 2 (4.0) | 3 (6.3) | 2 (4.1) | 5 (5.2) |
| Dyspepsia | - | 1 (4.0) | 1 (2.0) | 3 (6.3) | 2 (4.1) | 5 (5.2) |
| Nausea | 2 (8.0) | - | 2 (4.0) | 2 (4.2) | 1 (2.0) | 3 (3.1) |
| Vomiting | 1 (4.0) | 1 (4.0) | 2 (4.0) | 1 (2.1) | 2 (4.1) | 3 (3.1) |
| Constipation | - | 2 (8.0) | 2 (4.0) | 1 (2.1) | 1 (2.0) | 2 (2.1) |
| Abdominal pain | - | 1 (4.0) | 1 (2.0) | 1 (2.1) | 1 (2.0) | 2 (2.1) |
| **Respiratory, thoracic and mediastinal disorders** | 2 (8.0) | 4 (16.0) | 6 (12.0) | 8 (16.7) | 6 (12.2) | 14 (14.4) |
| Cough | 1 (4.0) | 2 (8.0) | 3 (6.0) | 3 (6.3) | 4 (8.2) | 7 (7.2) |
| Dyspnoea | - | - | - | 3 (6.3) | - | 3 (3.1) |
| Rhinorrhoea | - | 1 (4.0) | 1 (2.0) | 1 (2.1) | 1 (2.0) | 2 (2.1) |
| **Nervous system disorders** | 2 (8.0) | 3 (12.0) | 5 (10.0) | 4 (8.3) | 9 (18.4) | 13 (13.4) |
| Headache | - | 1 (4.0) | 1 (2.0) | 3 (6.3) | 3 (6.1) | 6 (6.2) |
| Dizziness | 1 (4.0) | 1 (4.0) | 2 (4.0) | 1 (2.1) | 1 (2.0) | 2 (2.1) |
| **Infections and infestations** | 3 (12.0) | 2 (8.0) | 5 (10.0) | 7 (14.6) | 7 (14.3) | 14 (14.4) |
| Nasopharyngitis | - | - | - | - | 4 (8.2) | 4 (4.1) |
| Bronchitis | 1 (4.0) | - | 1 (2.0) | 1 (2.1) | 1 (2.0) | 2 (2.1) |
| **General disorders and administration site conditions** | 4 (16.0) | 4 (16.0) | 8 (16.0) | 9 (18.8) | 7 (14.3) | 16 (16.5) |
| Asthenia | - | - | - | 3 (6.3) | 1 (2.0) | 4 (4.1) |
| Fatigue | 1 (4.0) | 3 (12.0) | 4 (8.0) | 2(4.2) | 1 (2.0) | 3 (3.1) |
| Oedema peripheral | 1 (4.0) | 1 (4.0) | 2 (4.0) | 1 (2.1) | 1 (2.0) | 2 (2.1) |
| Condition aggravated | 1 (4.0) | - | 1 (2.0) | - | 2 (4.1) | 2 (2.1) |
| Pyrexia | - | - | - | 1 (2.1) | 1 (2.0) | 2 (2.1) |
| **Metabolism and nutrition disorders** | 2 (8.0) | - | 2 (4.0) | 1 (2.1) | 3 (6.1) | 4 (4.1) |
| Decreased appetite | - | - | - | 1 (2.1) | 2 (4.1) | 3 (3.1) |
| Investigations | 2 (8.0) | 5 (20.0) | 7 (14.0) | 5 (10.4) | 10 (20.4) | 15 (15.5) |
| Weight decreased | - | - | - | 2(4.2) | 1 (2.0) | 3 (3.1) |
| **Musculoskeletal and connective tissue disorders** | 4 (16.0) | 2 (8.0) | 6 (12.0) | 2(4.2) | 3 (6.1) | 5 (5.2) |
| Arthralgia | - | 1 (4.0) | 1 (2.0) | - | 2 (4.1) | 2 (2.1) |

### 3.2.3.2 Results regarding Safety/Tolerability of the PDE4-inhibitor of formula III within the "AF-group" (separated by type of antifibrotic background treatment)

If you compare the results with regard to the frequency of the AE "gastrointestinal disorders" - and in particular with regard to the frequency of the AE "diarrhoea" - for the complete "AF-group" (as shown in Table 11) on the one hand and for that part of the "AF-group" that had obtained nintedanib as antifibrotic background medication ("nintedanib background medication group") and for that part of the "AF-group" that had obtained pirfenidone as antifibrotic background medication ("pirfenidone background medication group") on the other hand (as shown in Table 14), it becomes evident that the "pirfenidone background medication group" shows a clearly lower frequency of the AE "gastrointestinal disorders":
- in the "Comp. of form. III-arm" for the complete "AF-group" **36.7%** of the patients experienced a gastrointestinal disorder (see Table 11)
- in the "Comp. of form. III-arm" for the "nintedanib background medication sub-group" **46.2%** of the patients experienced a gastrointestinal disorder (see Table 14) and
   in the "Comp. of form. III-arm" for the "pirfenidone background medication sub-group" only **26.1%** of the patients experienced a gastrointestinal disorder (see Table 14)

Comparable results could be found from the respective "Placebo groups" of the "AF-group":
- in the "Placebo arm" for the complete "AF-group" **32.0%** of the patients experienced a gastrointestinal disorder (see Table 11)
- in the "Placebo-arm" for the "nintedanib background medication sub-group" **41.2%** of the patients experienced a gastrointestinal disorder (see Table 14)
   and
   in the "Placebo-arm" for the "pirfenidone background medication sub-group" only **12.5%** of the patients experienced a gastrointestinal disorder (see Table 14).

The very same trend can be observed, if you analyze the situation for the AE "diarrhoea" for the "Comp. of form. III - arm":
- in the "Comp. of form. III-arm" for the complete "AF-group" **30.6%** of the patients experienced diarrhoea (see Table 11)
- in the "Comp. of form. III-arm" for the "nintedanib background medication sub-group" **46.2%** of the patients experienced diarrhoea (see Table 14)
   and
   in the "Comp. of form. III-arm" for the "pirfenidone background medication sub-group" only **13.0%** of the patients experienced a diarrhoea (see Table 14)
   and for the respective "Placebo arm":
- in the "Placebo arm" for the complete "AF-group" **16.0%** of the patients experienced diarrhoea (see Table 11)
- in the "Placebo-arm" for the "nintedanib background medication sub-group" **23.5%** of the patients experienced diarrhoea (see Table 14)
   and
   in the "Placebo-arm" for the "pirfenidone background medication sub-group" even **0%** of the patients experienced diarrhoea (see Table 14).

This trend of a lower tendency to the AE gastrointestinal disorders, in particular to the AE diarrhoea, in case that pirfenidone is used as antifibrotic background medication (compared to nintedanib as antifibrotic background medication) is also supported from Table 15, wherein the frequencies/percentages of adverse events leading to a discontinuation of the trial medication is shown. From Table 15 it is evident that within the "AF-group" in the "active agent arm" ("Compound of Formula III") the combination of 18 mg b.i.d. of the compound of formula III with nintedanib as background medication leads to
- 4 (15.4 %) discontinuations of the trial treatment due to the AE gastrointestinal disorders
   and
- 3 (11.5 %) discontinuations of the trial treatment due to the AE of diarrhoea,
whereas the combination of 18 mg b.i.d. of the compound of formula III with pirfenidone as background medication leads to no discontinuations of the trial treatment at all (see Table 15). In all treatment arms, wherein pirfenidone was used as background medication (that means in the respective "active agent group" and "Placebo group") no single case of discontinuation of the trial treatment due to any gastrointestinal AEs was recorded (see Table 15). Therefore the combination treatment with 18 mg b.i.d. of the compound of formula III and pirfenidone (in doses as authorized) as background treatment might be associated with a reduced frequency and a reduced severity of the AE gastrointestinal disorder, in particular to a a reduced frequency and a reduced severity of the AE diarrhoea in comparison to the combination treatment with 18 mg b.i.d. of the compound of formula III and nintedanib (in doses as authorized) as background treatment.

**Table 12: Summary of Adverse Events within "AF-group" with background medication by nintedanib:**

| **"AF-group": Antifibrotic background medication with nintedanib** | | | | | |
|---|---|---|---|---|---|
| | | **Placebo** | | **Comp. of form. III** | |
| | | N | % | N | % |
| Number of patients | | 17 | 100.0 | 26 | 100.0 |
| Patients with any AE | | 13 | 76.5 | 21 | 80.8 |
| Patients with severe AEs | | 1 | 5.9 | 1 | 3.8 |
| Patients with investigator defined drug related AEs | | 4 | 23.5 | 12 | 46.2 |
| Patients with AEs leading to discontinuation of the trial drug | | 0 | 0 | 7 | 26.9 |
| Patients with pre-specified AEs of special interest | | 0 | 0 | 0 | 0 |
| Patients with serious AEs | | 0 | 0 | 1 | 3.8 |
| | Results in Death | 0 | 0 | 0 | 0 |
| | Immediately life threatening | 0 | 0 | 0 | 0 |
| | Persistent or significant disability | 0 | 0 | 0 | 0 |
| | Requires or prolongs hospitalization | 0 | 0 | 1 | 3.8 |
| | Congenital anomaly/birth defect | 0 | 0 | 0 | 0 |
| | Other comparable medical criteria | 0 | 0 | 0 | 0 |
| Patients with other significant AEs | | 0 | 0 | 7 | 26.9 |

**Table 13: Summary of Adverse Events within "AF-group" with background medication by pirfenidone:**

| **"AF-group": Antifibrotic background medication with pirfenidone** | | | | | |
|---|---|---|---|---|---|
| | | **Placebo** | | **Comp. of form. III** | |
| | | N | % | N | % |
| Number of patients | | 8 | 100.0 | 23 | 100.0 |
| Patients with any AE | | 4 | 50.0 | 15 | 65.2 |
| Patients with severe AEs | | 0 | 0 | 1 | 4.3 |
| Patients with investigator defined drug related AEs | | 1 | 12.5 | 6 | 26.1 |
| Patients with AEs leading to discontinuation of the trial drug | | 0 | 0 | 3 | 13.0 |
| Patients with pre-specified AEs of special interest | | 0 | 0 | 1 | 4.3 |
| Patients with serious AEs | | 0 | 0 | 2 | 8.7 |
| | Results in Death | 0 | 0 | 1 | 4.3 |
| | Immediately life threatening | 0 | 0 | 0 | 0 |
| | Persistent or significant disability | 0 | 0 | 0 | 0 |
| | Requires or prolongs hospitalization | 0 | 0 | 2 | 8.7 |
| | Congenital anomaly/birth defect | 0 | 0 | 0 | 0 |
| | Other comparable medical criteria | 0 | 0 | 0 | 0 |
| Patients with other significant AEs | | 0 | 0 | 1 | 4.3 |

**Table 14: Frequently reported Adverse Events (AEs) during the clinical trial**

| | **Placebo** | | | | **Compound of Formula III** | | | |
|---|---|---|---|---|---|---|---|---|
| | **Non-AF** | **AF Ninted.** | **AF Pirf.** | **Total** | **Non-AF** | **AF Ninted.** | **AF Pirf.** | **Total** |
| | **N=25, n(%)** | **N=17, n(%)** | **N=8, n(%)** | **N=50, n(%)** | **N=48, n(%)** | **N=26, n(%)** | **N=15, n(%)** | **N=97 N(%)** |
| **Gastrointestinal disorders** | 4 (16.0) | 7 **(41.2)** | 1 **(12.5)** | 12 (24.0) | 13 (27.1) | 12 **(46.2)** | 6 **(26.1)** | 31(32.0) |
| Diarrhoea | 2(8.0) | 4 **(23.5)** | - | 6 (12.0) | 8 (16.7) | 12 **(46.2)** | 3 **(13.0)** | 23 (23.7) |
| Flatulence | 1 (4.0) | 1 (5.9) | - | 2 (4.0) | 3 (6.3) | 1 (3.8) | 1 (4.3) | 5 (5.2) |
| Dyspepsia | - | 1 (5.9) | - | 1 (2.0) | 3 (6.3) | 1 (3.8) | 1 (4.3) | 5 (5.2) |
| Nausea | 2 (8.0) | - | - | 2 (4.0) | 2 (4.2) | - | 1 (4.3) | 3 (3.1) |
| Vomiting | 1 (4.0) | 1 (5.9) | - | 2 (4.0) | 1 (2.1) | 1 (3.8) | 1 (4.3) | 3 (3.1) |
| Constipation | - | 1 (5.9) | 1 (12.5) | 2 (4.0) | 1 (2.1) | - | 1 (4.3) | 2 (3.1) |
| Abdominal Pain | - | - | 1 (12.5) | 1 (2.0) | 1 (2.1) | - | 1 (4.3) | 2 (2.1) |
| **Respiratory, thoracic and mediastinal disorders** | 2 (8.0) | 3 (17.6) | 1 (12.5) | 6 (12.0) | 8 (16.7) | 3 (11.5) | 3 (13.0) | 14 (14.4) |
| Cough | 1 (4.0) | 1 (5.9) | 1 (12.5) | 3 (6.0) | 3 (6.3) | 2 (7.7) | 2 (8.7) | 7 (7.2) |
| Dyspnoea | - | - | - | - | 3 (6.3) | - | - | 3 (3.1) |
| Rhinorrhoea | - | 1 (5.9) | - | 1 (2.0) | 1 (2.1) | - | 1 (4.3) | 2 (2.1) |
| **Nervous system disorders** | 2 (8.0) | 3 (17.6) | - | 5 (10.0) | 4 (8.3) | 4 (15.4) | 5 (21.7) | 13 (13.4) |
| Headache | - | 1 (5.9) | - | 1 (2.0) | 3 (6.3) | 2 (7.7) | 1 (4.3) | 6 (6.2) |
| Dizziness | 1 (4.0) | 1 (5.9) | - | 2 (4.0) | 1 (2.1) | - | 1 (4.3) | 2 (2.1) |
| **Infections and infestations** | 3 (12.0) | 1 (5.9) | 1 (12.5) | 5 (101.0) | 7 (14.6) | 6 (23.1) | 1 (4.3) | 14 (14.4) |
| Nasopharyngitis | - | - | - | - | - | 4 (15.4) | - | 4 (4.1) |
| Bronchitis | 1 (4.0) | - | - | 1 (2.0) | 1 (2.1) | 1 (3.8) | - | 2 (2.1) |
| **General Disorders and Administration Side conditions** | 4 (16.0) | 3 (17.6) | 1 (12.5) | 8 (16.0) | 9 (18.8) | 5 (19.2) | 2 (8.7) | 16 (16.5) |
| Asthenia | - | - | - | - | 3 (6.3) | - | 1 (4.3) | 4 (4.1) |
| Fatigue | 1 (4.0) | 1 (11.8) | 1 (12.5) | 4 (8.0) | 2 (4.2) | 1 (3.8) | 1 (4.3) | 3 (3.1) |
| Oedema peripheral | 1 (4.0) | 1 (5.9) | - | 2 (4.0) | 1 (2.1) | 1 (3.8) | - | 2 (2.1) |
| Condition aggravated | 1 (4.0) | - | - | 1 (2.0) | - | 1 (3.8) | 1 (4.3) | 2 (2.1) |
| Pyrexia | - | - | - | - | 1 (2.1) | 1 (2.1) | - | 2 (2.1) |
| **Metabolism and nutrition disorders** | 2 (8.0) | - | - | 2 (4.0) | 1 (2.1) | 3 (11.5) | - | 4 (4.1) |
| Decreased appetite | - | - | - | - | 1 (2.1) | 2 (7.7) | - | 3 (3.1) |
| Investigations | 2 (8.0) | 3 (17.6) | 2 (25.0) | 7 (14.0) | 5 (10.4) | 7 (26.9) | 3 (13.0) | 15 (15.5) |
| Weight decreased | - | - | - | - | 2 (4.2) | 1 (3.8) | - | 3 (3.1) |
| **Musculoskeletal and connective tissue disorders** | 4 (16.0) | 1 (5.9) | 1 (12.5) | 6 (12.0) | 2 (4.2) | 2 (7.7) | 1 (4.3) | 5 (5.2) |
| Arthralgia | - | 1 (5.9) | - | 1 (2.0) | - | 2 (7.7) | - | 2 (2.1) |

**Table 15: Gastrointestinal disorders leading to discontinuation of trial treatment**

| | **Placebo** | | | | **Compound of Formula III** | | | |
|---|---|---|---|---|---|---|---|---|
| | **Non-AF** | **AF Ninted.** | **AF Pirf.** | **Total** | **Non-AF** | **AF Ninted.** | **AF Pirf.** | **Total** |
| | **N=25, n(%)** | **N=17, n(%)** | **N=8, n(%)** | **N=50, n(%)** | **N=48, n(%)** | **N=26, n(%)** | **N=15, n(%)** | **N=97 N(%)** |
| **Gastrointestinal disorders** | - | - | - | - | 1 (2.1) | 4 (15.4) | - | 5 (5.2) |
| Diarrhoea | - | - | - | - | - | 3 (11.5) | - | 3 (3.1) |
| Dyspepsia | - | - | - | - | - | 1 (3.8) | - | 1 (1.0) |
| Vomiting | | | | | 1 (2.1) | - | - | 1 (1.0) |

### 4. Conclusion

The results of the "Non-AF-group" of the clinical trial support the hypothesis that the PDE4-inhibitor of formula III administered twice daily (b.i.d.) in the dose of 18 mg without antifibrotic background medication substantially slows down FVC decline in IPF-patients for the treatment period of 12 weeks ("Non-AF-group": for the "Comp. of form. III (= active agent)-group the change from baseline in FVC after 12 weeks is + 6.1 ml; for the respective "Placebo-group" the change from baseline in FVC after 12 weeks is -95.6 ml, see Table 5, Fig. 1).

For the "Non-AF-group" this leads to an adjusted mean difference between changes from baseline in FVC to week 12 between the "active agent group" and the "Placebo group" of 101.72 ml which supports the hypothesis that the PDE4-inhibitor of formula III administered in a dose of 18 mg twice daily to PF-ILD/IPF patients without antifibotic background treatment shows a high therapeutic efficacy for the treatment of PF-ILD patients, preferably of IPF patients (see Table 5, Fig. 1).

On the other hand only 1 patient (2.1 %) of the "active agent group" compared to no patient (0%) of the "placebo-group" of the "Non-AF-arm" experienced a gastrointestinal disorder leading to a discontinuation of the trial (see Table 15).

The results of that part of the "AF-group that had obtained nintedanib as antifibrotic background medication" support the hypothesis that the PDE4-inhibitor of formula III administered twice daily (b.i.d.) in the dose of 18 mg in combination with nintedanib as antifibrotic background substantially slows down FVC decline in IPF-patients for the treatment period of 12 weeks compared to the treatment with nintedanib alone ("AF-group with nintedanib as background medication": for the "Comp. of form. III (= active agent)-group" the change from baseline in FVC after 12 weeks is + 23.41 ml; for the respective "Placebo-group" the change from baseline in FVC after 12 weeks is -82.01 ml (see Table 7, Fig. 3)).

This results in an adjusted mean difference between changes from baseline in FVC to week 12 between the "active agent group" and the "Placebo group" of 105.43 ml for the "AF-group that had obtained nintedanib as antifibrotic background medication" (see Table 7, Fig. 3).

This supports the hypothesis that the combination of 18 mg of the PDE4-inhibitor of formula III administered twice daily and of nintedanib (administered in doses and dose regimens as authorized) may lead to an extraordinarily high therapeutic efficacy.

On the other hand, 4 patients (15.4 %) of the "active agent group" compared to no patient (0%) of the "placebo-group" of the "AF-arm with nintedanib as antifibrotic background medication" experienced a gastrointestinal disorder as AE leading to a discontinuation of the trial treatment, 3 of these 4 patients experienced the AE diarrhoea leading to a discontinuation of the trial treatment (see Table 15).

The results of the "AF-group that had obtained pirfenidone as antifibrotic background medication" support the hypothesis that 18 mg of the PDE4-inhibitor of formula III administered twice daily (b.i.d.) in combination with pirfenidone as antifibrotic background medication substantially slows down FVC decline in IPF-patients for the treatment period of 12 weeks compared to the treatment with pirfenidone alone ("AF-group with pirfenidone as background medication": for the "Comp. of form. III (= active agent)-group" the change from baseline in FVC after 12 weeks is - 18.67 ml; for the respective "Placebo-group" the change from baseline in FVC after 12 weeks is -80.06 ml (see Table 7, Fig. 4)).

This results in an adjusted mean difference between changes from baseline in FVC to week 12 between the "active agent group" and the "Placebo group" of 61.30 ml for the "AF-group that had obtained pirfenidone as antifibrotic background medication" (see Table 7, Fig. 4).

On the other hand, not a single patient of the "active agent group" nor of the "placebo-group" of the "AF-arm with pirfenidone as antifibrotic background medication" experienced a gastrointestinal disorder leading to a discontinuation of the trial (see Table 15).

On the same time, the administration of 18 mg of the PDE4-inhibitor of formula III twice daily (b.i.d.) increased the frequency of the adverse event "gastrointestinal disorders" and in particular the frequency of the AE "diarrhoea" in all treatment arms (in the "Non-AF-group" and in the "AF-group").

However, within the "AF-group" the frequency and severity of the reported adverse events of gastrointestinal disorders - and in particular the frequency and severity of the reported adverse events of diarrhoea - were strongly dependent from the kind of antifibrotic background medication that was used. In IPF-patients that had obtained pirfenidone as antifibrotic background medication adverse events of gastrointestinal disorders, in particular adverse events of diarrhoea, were less frequently recorded and less frequently lead to the discontinuation of trial treatment (see Tables 14 and 15) compared to IPF-patients that had obtained nintedanib as antifibrotic background treatment.

In IPF-patients that had obtained nintedanib as antifibrotic background medication adverse events of gastrointestinal disorders, in particular adverse events of diarrhoea, were recorded more frequently and in some cases also lead to the discontinuation of trial treatment (see Tables 14 and 15). However, the IPF-patients that had obtained nintedanib as antifibrotic background medication showed better therapeutic efficacy than the IPF-patients that had obtained pirfenidone as antifibrotic background treatment.

Consequently, the results for the "AF-group with nintedanib as antifibrotic background treatment" of the presented phase II trial support the hypothesis that the PDE4-inhibitor of formula III in the dose of 18 mg twice daily combined with nintedanib background treatment (in doses and dose regimens as authorized for nintedanib (preferably 150 mg b.i.d. or 100 mg b.i.d.) may lead to an excellent therapeutic efficacy combined with an acceptable tolerability and safety profile (leading to an acceptable patient compliance). The combination of nintedanib and of the PDE4-inhibitor of formula III in the dose of 18 mg administered twice daily might therefore in particular be suitable for PF-ILD/IPF-patients that have no tendency or predisposition to gastrointestinal disorders, in particular to diarrhoea, or that have experienced in their PF-ILD/IPF treatment history so far no problems due to gastrointestinal disorders, in particular due to diarrhoea.

Consequently, the results for the "AF-group with pirfenidone as antifibrotic background treatment" of the presented phase II trial support the hypothesis that the PDE4-inhibitor of formula III in the dose of 18 mg twice daily combined with pirfenidone background treatment (in doses and dose regimens as authorized for pirfenidone (preferably in doses and dose regimens leading to a daily dose between 801 mg and 2403 mg) may lead to a satisfying therapeutic efficacy combined with an excellent tolerability and safety profile (leading to an excellent compliance of the patient). The combination of pirfenidone and of the PDE4-inhibitor of formula III in the dose of 18 mg administered twice daily might therefore in particular be suitable for PF-ILD/IPF-patients that have a predisposition or tendency to gastrointestinal disorders, in particular to diarrhoea, or that have experienced problems in their PF-ILD/IPF treatment history so far due to gastrointestinal disorders, in particular due to diarrhoea.

## Claims

1. An oral pharmaceutical composition comprising
• 18 mg of the PDE4B-inhibitor of formula III 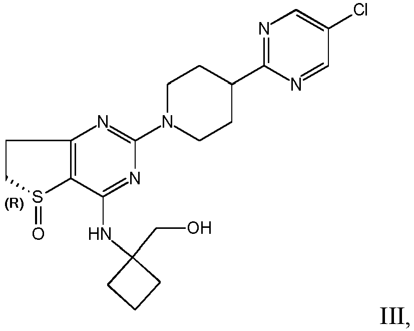
• a therapeutically effective dose of pirfenidone and
• optionally, one or more pharmaceutically acceptable carriers or excipients for use in a method of treating a patient suffering from a progressive fibrosing interstitial lung disease (PF-ILD), wherein this oral pharmaceutical composition is administered to said patient twice daily.

2. The oral pharmaceutical composition for use in a method of treating a patient suffering from progressive fibrosing interstitial lung disease (PF-ILD) according to claim 1, wherein the progressive fibrosing interstitial lung disease (PF-ILD) is idiopathic pulmonary fibrosis (IPF).

3. The oral pharmaceutical composition for use in a method of treating a patient suffering from progressive fibrosing interstitial lung disease (PF-ILD) according to at least one of claims 1 or 2, wherein the therapeutically effective dose of pirfenidone sums up to a daily dose between 801 mg and 2403 mg.

4. An oral pharmaceutical composition comprising the PDE4B-inhibitor of formula III 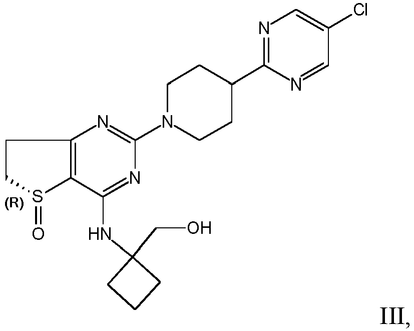 in the dose of 18 mg for use in a method of treating a patient suffering from one or more Progressive Fibrosing Interstitial Lung Diseases (PF-ILDs), wherein said oral pharmaceutical composition comprising 18 mg of the PDE4B-inhibitor of formula III is administered twice daily in combination with a separate oral pharmaceutical composition comprising a therapeutically effective amount of pirfenidone.

5. The oral pharmaceutical composition comprising the PDE4B-inhibitor of formula III in the dose of 18 mg for use in a method for treating a patient suffering from one or more Progressive Fibrosing Interstitial Lung Diseases (PF-ILDs) according to claim 4, wherein the one or more Progressive Fibrosing Interstitial Lung Disease (PF-ILDs) is idiopathic pulmonary fibrosis (IPF).

6. The oral pharmaceutical composition comprising the PDE4B-inhibitor of formula III in the dose of 18 mg for use in a method for treating a patient suffering from one or more Progressive Fibrosing Interstitial Lung Diseases (PF-ILDs) according to at least one of claims 4 or 5, wherein the therapeutically effective amount of pirfenidone is administered in a dose and dose regimen summing up to a daily dose between 801 mg and 2403 mg.

7. A kit for use in a method of treating a patient suffering from a Progressive Fibrosing Interstitial Lung Disease (PF-ILD) comprising:
• a first oral pharmaceutical composition or dosage form comprising 18 mg of the PDE4B-inhibitor of formula III 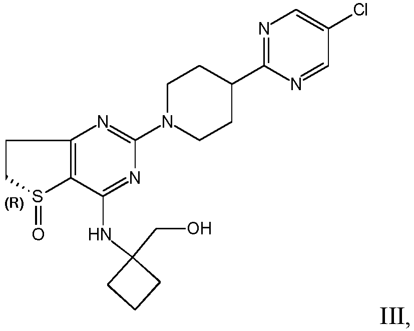 and optionally, one or more pharmaceutically acceptable carriers and/or excipients and
• a second oral pharmaceutical composition or dosage form comprising pirfenidone in a therapeutically effective dose and optionally, one or more pharmaceutically acceptable carriers and/or excipients,
wherein the first pharmaceutical composition or dosage form is to be administered twice daily.

8. The kit for use in a method of treating a patient suffering from a Progressive Fibrosing Interstitial Lung Disease (PF-ILD) according to claim 7, wherein the Progressive Fibrosing Interstitial Lung Disease (PF-ILD) is idiopathic pulmonary fibrosis (IPF).

9. The kit for use in a method of treating a patient suffering from a Progressive Fibrosing Interstitial Lung Disease (PF-ILD) according to one of claims 7 or 8, wherein the first pharmaceutical composition or dosage form is to be administered simultaneously, concurrently, sequentially, successively, alternately or separately with the second pharmaceutical composition or dosage form.

10. The kit for use in a method of treating a patient suffering from a Progressive Fibrosing Interstitial Lung Disease (PF-ILD) according to at least one of claims 7 to 9, wherein the second pharmaceutical composition or dosage form comprises pirfenidone in the dose of either 267 mg and is administered three times daily or in a dose of 534 mg and is administered three times daily or in the dose of 801 mg and is administered three times daily.

11. The kit for use in a method of treating a patient suffering from a Progressive Fibrosing Interstitial Lung Disease (PF-ILD) according to at least one of claims 7 to 10, wherein the second pharmaceutical composition or dosage form comprises pirfenidone in a daily dose between 801 mg and 2403 mg.

## Patentansprüche

1. Orale pharmazeutische Zusammensetzung, umfassend
• 18 mg des PDE4B-Hemmers nach Formel III 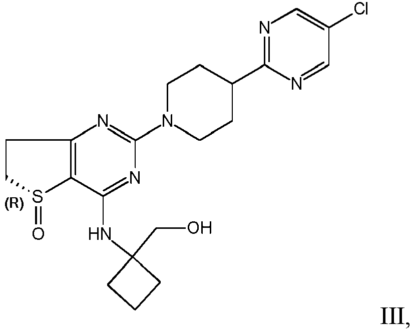
• eine therapeutisch wirksame Dosis an Pirfenidon sowie
• möglicherweise einen oder mehrere pharmazeutisch unbedenkliche Träger oder Hilfsstoffe zur Verwendung in einem Verfahren zur Behandlung eines Patienten, der unter einer fortschreitenden fibrosierenden interstitiellen Lungenerkrankung (PF-ILD) leidet, wobei diese orale pharmazeutische Zusammensetzung dem Patienten zweimal täglich verabreicht wird.

2. Orale pharmazeutische Zusammensetzung zur Verwendung in einem Verfahren zur Behandlung eines Patienten, der unter fortschreitender fibrosierender interstitieller Lungenerkrankung (PF-ILD) leidet, gemäß Anspruch 1, wobei es sich bei der fortschreitenden fibrosierenden interstitiellen Lungenerkrankung (PF-ILD) um idiopathische Lungenfibrose (IPF) handelt.

3. Orale pharmazeutische Zusammensetzung zur Verwendung in einem Verfahren zur Behandlung eines Patienten, der unter fortschreitender fibrosierender interstitieller Lungenerkrankung (PF-ILD) leidet, gemäß mindestens einem der Ansprüche 1 oder 2, wobei die therapeutisch wirksame Dosis an Pirfenidon sich auf eine Tagesgesamtdosis zwischen 801 mg und 2403 mg beläuft.

4. Orale pharmazeutische Zusammensetzung, welche den PDE4B-Hemmer nach Formel III 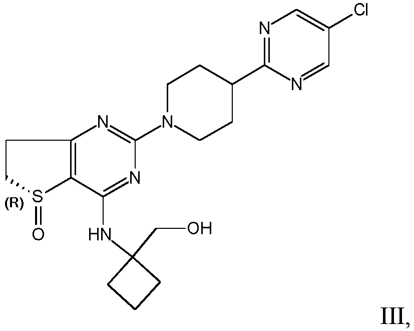 in der Dosis von 18 mg umfasst, zur Verwendung in einem Verfahren zur Behandlung eines Patienten, der unter einer oder mehreren fortschreitenden fibrosierenden interstitiellen Lungenerkrankungen (PF-ILDs) leidet, wobei die orale pharmazeutische Zusammensetzung, welche 18 mg des PDE4B-Hemmers nach Formel III umfasst, zweimal täglich in Kombination mit einer gesonderten oralen pharmazeutischen Zusammensetzung verabreicht wird, welche eine therapeutisch wirksame Menge an Pirfenidon umfasst.

5. Orale pharmazeutische Zusammensetzung, welche den PDE4B-Hemmer nach Formel III in der Dosis von 18 mg umfasst, zur Verwendung in einem Verfahren zur Behandlung eines Patienten, der unter einer oder mehreren fortschreitenden fibrosierenden interstitiellen Lungenerkrankungen (PF-ILDs) leidet, gemäß Anspruch 4, wobei es sich bei der einen oder den mehreren fortschreitenden fibrosierenden interstitiellen Lungenerkrankungen (PF-ILDs) um idiopathische Lungenfibrose (IPF) handelt.

6. Orale pharmazeutische Zusammensetzung, welche den PDE4B-Hemmer nach Formel III in der Dosis von 18 mg umfasst, zur Verwendung in einem Verfahren zur Behandlung eines Patienten, der unter einer oder mehreren fortschreitenden fibrosierenden interstitiellen Lungenerkrankungen (PF-ILDs) leidet, gemäß mindestens einem der Ansprüche 4 oder 5, wobei die therapeutisch wirksame Dosis an Pirfenidon in einer Dosis verabreicht wird und sich das Dosierschema auf eine Tagesgesamtdosis zwischen 801 mg und 2403 mg beläuft.

7. Kit zur Verwendung in einem Verfahren zur Behandlung eines Patienten, der unter einer fortschreitenden fibrosierenden interstitiellen Lungenerkrankung (PF-ILD) leidet, wobei es Folgendes umfasst:
• eine erste orale pharmazeutische Zusammensetzung oder Darreichungsform, die 18 mg des PDE4B-Hemmers nach Formel III 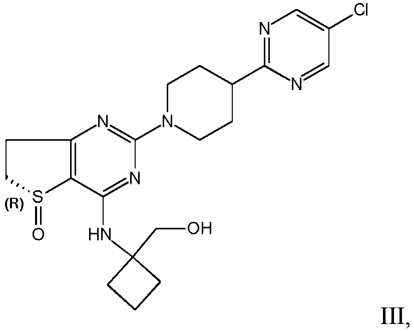 und möglicherweise einen oder mehrere pharmazeutisch unbedenkliche Träger und/oder Hilfsstoffe umfasst, und
• eine zweite orale pharmazeutische Zusammensetzung oder Darreichungsform, die Pirfenidon in einer therapeutisch wirksamen Dosis und möglicherweise einen oder mehrere pharmazeutisch unbedenkliche Träger und/oder Hilfsstoffe umfasst,
wobei die erste pharmazeutische Zusammensetzung oder Darreichungsform zweimal täglich zu verabreichen ist.

8. Kit zur Verwendung in einem Verfahren zur Behandlung eines Patienten, der unter einer fortschreitenden fibrosierenden interstitiellen Lungenerkrankung (PF-ILD) leidet, gemäß Anspruch 7, wobei es sich bei der fortschreitenden fibrosierenden interstitiellen Lungenerkrankung (PF-ILD) um idiopathische Lungenfibrose (IPF) handelt.

9. Kit zur Verwendung in einem Verfahren zur Behandlung eines Patienten, der unter einer fortschreitenden fibrosierenden interstitiellen Lungenerkrankung (PF-ILD) leidet, gemäß einem der Ansprüche 7 oder 8, wobei die erste pharmazeutische Zusammensetzung oder Darreichungsform bezüglich der zweiten pharmazeutischen Zusammensetzung oder Darreichungsform gleichzeitig, parallel, nacheinander, darauffolgend, im Wechsel mit dieser oder getrennt von dieser zu verabreichen ist.

10. Kit zur Verwendung in einem Verfahren zur Behandlung eines Patienten, der unter einer fortschreitenden fibrosierenden interstitiellen Lungenerkrankung (PF-ILD) leidet, gemäß mindestens einem der Ansprüche 7 bis 9, wobei die zweite pharmazeutische Zusammensetzung oder Darreichungsform Pirfenidon entweder in der Dosis von 267 mg mit dreimal täglicher Verabreichung oder in einer Dosis von 534 mg mit dreimal täglicher Verabreichung oder in der Dosis von 801 mg mit dreimal täglicher Verabreichung umfasst.

11. Kit zur Verwendung in einem Verfahren zur Behandlung eines Patienten, der unter einer fortschreitenden fibrosierenden interstitiellen Lungenerkrankung (PF-ILD) leidet, gemäß mindestens einem der Ansprüche 7 bis 10, wobei die zweite pharmazeutische Zusammensetzung oder Darreichungsform Pirfenidon in einer Tagesdosis zwischen 801 mg und 2403 mg umfasst.

## Revendications

1. Composition pharmaceutique orale comprenant
• 18 mg de l'inhibiteur de PDE4B de formule III 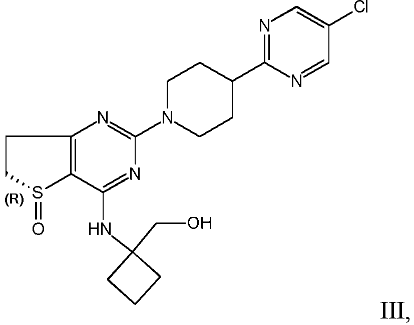
• une dose thérapeutiquement efficace de pirfénidone et
• éventuellement, un ou plusieurs supports ou excipients pharmaceutiquement acceptables pour une utilisation dans un procédé de traitement d'un patient souffrant d'une maladie pulmonaire interstitielle fibrosante progressive (PF-ILD), dans laquelle cette composition pharmaceutique orale est administrée audit patient deux fois par jour.

2. Composition pharmaceutique orale pour une utilisation dans un procédé de traitement d'un patient souffrant d'une maladie pulmonaire interstitielle fibrosante progressive (PF-ILD) selon la revendication 1, dans laquelle la maladie pulmonaire interstitielle fibrosante progressive (PF-ILD) est une fibrose pulmonaire idiopathique (IPF).

3. Composition pharmaceutique orale pour une utilisation dans un procédé de traitement d'un patient souffrant d'une maladie pulmonaire interstitielle fibrosante progressive (PF-ILD) selon au moins l'une des revendications 1 ou 2, dans laquelle la dose thérapeutiquement efficace de pirfénidone est égale à une dose quotidienne comprise entre 801 mg et 2403 mg.

4. Composition pharmaceutique orale comprenant l'inhibiteur de PDE4B de formule III 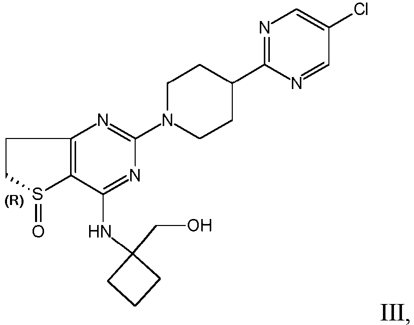 en la dose de 18 mg pour une utilisation dans un procédé de traitement d'un patient souffrant d'une ou plusieurs maladies pulmonaires interstitielles fibrosantes progressives (PF-ILD), dans laquelle ladite composition pharmaceutique orale comprenant 18 mg de l'inhibiteur de PDE4B de formule III est administrée deux fois par jour en combinaison avec une composition pharmaceutique orale séparée comprenant une quantité thérapeutiquement efficace de pirfénidone.

5. Composition pharmaceutique orale comprenant l'inhibiteur de PDE4B de formule III en la dose de 18 mg pour une utilisation dans un procédé de traitement d'un patient souffrant d'une ou plusieurs maladies pulmonaires interstitielles fibrosantes progressives (PF-ILD) selon la revendication 4, dans laquelle une ou plusieurs maladies pulmonaires interstitielles fibrosantes progressives (PF-ILD) sont une fibrose pulmonaire idiopathique (IPF).

6. Composition pharmaceutique orale comprenant l'inhibiteur de PDE4B de formule III en la dose de 18 mg pour une utilisation dans un procédé de traitement d'un patient souffrant d'une ou plusieurs maladies pulmonaires interstitielles fibrosantes progressives (PF-ILD) selon au moins l'une des revendications 4 ou 5, dans laquelle la quantité thérapeutiquement efficace de pirfénidone est administrée en une dose et un schéma posologique totalisant jusqu'à une dose quotidienne comprise entre 801 mg et 2403 mg.

7. Kit pour une utilisation dans un procédé de traitement d'un patient souffrant d'une maladie pulmonaire interstitielle fibrosante progressive (PF-ILD) comprenant :
• une première composition pharmaceutique orale ou forme posologique comprenant 18 mg de l'inhibiteur de PDE4B de formule III 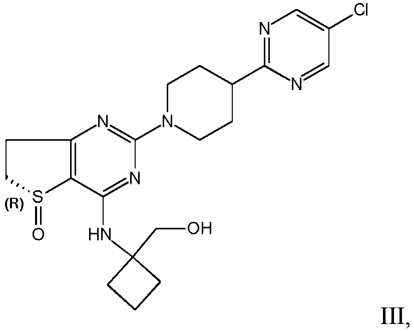 et éventuellement, un ou plusieurs supports et/ou excipients pharmaceutiquement acceptables et
• une seconde composition pharmaceutique orale ou forme posologique comprenant de la pirfénidone en une dose thérapeutiquement efficace et, éventuellement, un ou plusieurs supports et/ou excipients pharmaceutiquement acceptables,
dans lequel la première composition pharmaceutique ou forme posologique doit être administrée deux fois par jour.

8. Kit pour une utilisation dans un procédé de traitement d'un patient souffrant d'une maladie pulmonaire interstitielle fibrosante progressive (PF-ILD) selon la revendication 7, dans lequel la maladie pulmonaire interstitielle fibrosante progressive (PF-ILD) est une fibrose pulmonaire idiopathique (IPF).

9. Kit pour une utilisation dans un procédé de traitement d'un patient souffrant d'une maladie pulmonaire interstitielle fibrosante progressive (PF-ILD) selon l'une des revendications 7 ou 8, dans lequel la première composition pharmaceutique ou forme posologique doit être administrée simultanément, en parallèle, séquentiellement, successivement, alternativement ou séparément avec la seconde composition pharmaceutique ou forme posologique.

10. Kit pour une utilisation dans un procédé de traitement d'un patient souffrant d'une maladie pulmonaire interstitielle fibrosante progressive (PF-ILD) selon au moins l'une des revendications 7 à 9, dans lequel la seconde composition pharmaceutique ou forme posologique comprend de la pirfénidone en la dose de 267 mg et est administrée trois fois par jour ou en une dose de 534 mg et est administrée trois fois par jour ou en la dose de 801 mg et est administrée trois fois par jour.

11. Kit pour une utilisation dans un procédé de traitement d'un patient souffrant d'une maladie pulmonaire interstitielle fibrosante progressive (PF-ILD) selon au moins l'une des revendications 7 à 10, dans lequel la seconde composition pharmaceutique ou forme posologique comprend de la pirfénidone en une dose quotidienne comprise entre 801 mg et 2403 mg.
